# EUROPÄISCHE PATENTSCHRIFT

(11) **EP 2 616 424 B1**
(45) Veröffentlichungstag und Bekanntmachung des Hinweises auf die Patenterteilung: **26.08.2015**
(21) Anmeldenummer: 11754665.5
(22) Anmeldetag: 09.09.2011
(51) Int. Cl.: C07C 41/48, C07C 45/42, C07C 45/62, C07C 45/81, C07C 45/74, C07C 45/82, C07C 45/51, C07C 45/85, C25B 3/02

(54) **VERFAHREN ZUR HERSTELLUNG VON 2-METHYL-3-(4-TERT-BUTYLPHENYL)-PROPANAL MIT HOHER PARA-ISOMERENREINHEIT**
METHOD FOR PRODUCING 2-METHYL-3-(4-TERT-BUTYLPHENYL)-PROPANAL HAVING HIGH PARA-ISOMER PURITY
PROCÉDÉ DE PRODUCTION DE 2-MÉTHYL-3-(4-TERT-BUTYLPHÉNYL)-PROPANAL À PURETÉ ÉLEVÉE EN ISOMÈRE PARA

(30) Priorität: 16.09.2010 EP 10177074
(43) Veröffentlichungstag der Anmeldung: 24.07.2013
(73) Patentinhaber: BASF SE, 67056 Ludwigshafen (DE)
(72) Erfinder: GRIESBACH, Ulrich, 68305 Mannheim (DE); BOTZEM, Jörg, 67117 Limburgerhof (DE); STECKER, Florian, 68167 Mannheim (DE)
(86) Internationale Anmeldenummer: PCT/EP2011/065596
(87) Internationale Veröffentlichungsnummer: WO 2012/034930

(56) Entgegenhaltungen:
- EP-A1- 0 638 665
- WO-A1-2009/059944
- US-A- 2 875 131
- DATABASE CA [Online] CHEMICAL ABSTRACTS SERVICE, COLUMBUS, OHIO, US; BOSMA, COOS ET AL: "Anodic oxidation of 4-t-butyltoluene to 4-t-butylbenzaldehyde dimethyl acetal: optimization and scale-up", XP002666751, gefunden im STN Database accession no. 2000:100969 & BOSMA, COOS ET AL: "Anodic oxidation of 4-t-butyltoluene to 4-t-butylbenzaldehyde dimethyl acetal: optimization and scale-up", SOUTH AFRICAN JOURNAL OF CHEMISTRY , 52(4), 133-144 CODEN: SAJCDG; ISSN: 0379-4350, 1999,
- DATABASE Reaxys [Online] 1899, Moritz, Wolffenstein: XP002666752, & MORITZ, WOLFFENSTEIN: CHEM. BER., Bd. 32, 1899, Seite 2532,

## Beschreibung

Die vorliegende Erfindung betrifft ein Verfahren zur Herstellung von 2-Methyl-3-(4-tert-butylphenyl)-propanal mit hoher para-Isomerenreinheit sowie ein Verfahren zur Herstellung von 4-tert-Butylbenzaldehyd mit hoher para-Isomerenreinheit.

2-Methyl-3-(4-tert-butylphenyl)-propanal wird insbesondere als Maiglöckchen-Riechstoff eingesetzt und in diesem Zusammenhang häufig auch mit den geschützten Handelsnamen Lysmeral^{®}, Lilial^{®} oder Lilestralis^{®} bezeichnet. Auf Grund der eingesetzten Herstellverfahren enthält das kommerziell vertriebene 2-Methyl-3-(4-tert-butylphenyl)-propanal der Formel (I) immer auch Anteile an 2-Methyl-3-(3-tert-butylphenyl)-propanal der Formel (II).

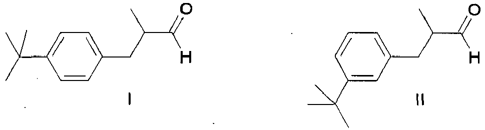

Es sind mehrere Verfahren bekannt, die einen Zugang zu Verbindung (I) oder Mischungen aus Verbindungen (I) und (II) ermöglichen. So lehrt die Schrift EP 0 045 571 einen Zugang zu den Verbindungen (I) und (II) ausgehend von Benzaldehyd durch Kondensation mit Propanal und nachfolgender Hydrierung des α,β-ungesättigten Aldehydes zu 2-Methyl-3-phenyl-1-propanol.

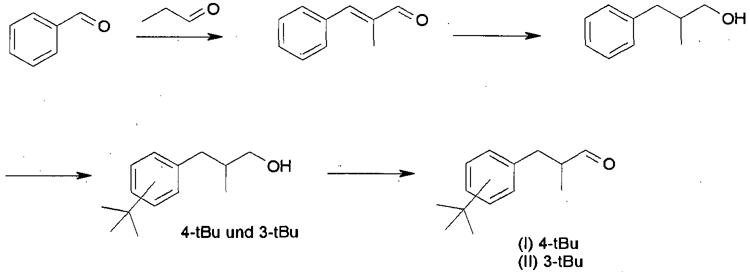

Durch Steuerung der Reaktionsbedingungen bei der Einführung des tert-Butylrestes an 2-Methyl-3-phenyl-1-propanol kann das Verhältnis zwischen dem entsprechenden para-Isomer und dem meta-Isomer in einem gewissen Umfang eingestellt werden. Die beiden isomeren Alkohole werden anschließend zu den Zielverbindungen (I) und (II) dehydriert.

Ein weiteres Verfahren zur Herstellung von 2-Methyl-3-(4-tert-butylphenyl)-propanal wird in WO 2001/027061 und der dort zitierten Literatur beschrieben.

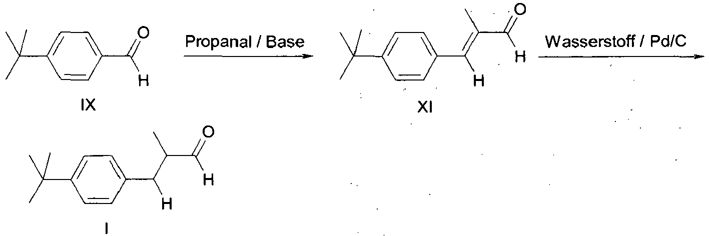

In EP 0 638 665 und der dort zitierten Literatur wird die Synthese des Diacetals der Formel VII beschrieben, welches durch Hydrolyse zu dem entsprechenden Aldehyd der Formel IX umgesetzt werden kann, wobei das Diacetal der Formel VII durch elektrochemische Oxidation von 4-tert-Butyltoluol der Formel III erhalten wird.

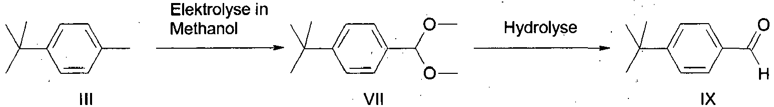

Das nach WO 2001/027061 hergestellte 2-Methyl-3-(4-tert-butylphenyl)-propanal enthält üblicherweise ca. 0,5 bis 2 Gew.-% des entsprechenden meta-Isomers der Formel II, nämlich 2-Methyl-3-(3-tert-butylphenyl)-propanal, bezogen auf die Gesamtmasse der Verbindungen der Formeln I und II, da das als Ausgangsprodukt eingesetzte 4-tert-Butyltoluol ca. 1 bis 5 Gew.-% 3-tert-Butyltoluol der Formel IV bezogen auf die Gesamtmasse der Verbindungen der Formeln III und IV enthält.

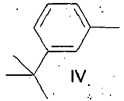

In DE 102 23 970 wird die destillative Aufreinigung von 2-Methyl-3-(4-tert-butylphenyl)-propanal der Formel I aus einem Rohgemisch mittels einer Trennwandkolonne beschrieben, wobei ein 2-Methyl-3-(4-tert-butylphenyl)-propanal mit einem Gehalt an Verunreinigungen von weniger als 1,0 Gew.-% isoliert wurde.

US 2875131 A beschreibt in Beispiel 1 die Herstellung von 2-Methyl-3-(4-tert-butylphenyl)-propanol ausgehend von p-tert-Butylbenzaldehyd, welches mit Propionaldehyd umgesetzt und anschließend hydrogeniert wird.

Bosma et al. (Bosma et al, S.Afr. J. Chem. 1999, 52(4), 133-144) beschreibt die Herstellung von 4-tert-Butyltoluol durch anodische Oxidation.

Moritz und Wolffenstein (Moritz und Wolffenstein, Chem. Ber. 1899, 32, 2531-2534) offenbart die Herstellung von 3-tert-Butylbenzaldehyd und dem entsprechenden Phenylhydrazon.

WO 2009/059944 A1 beschreibt ein Verfahren zur Herstellung von Benzaldehyddimethylacetalen durch eine anodische Methoxylierung von Benzylethern.

Bislang stellte die Anwesenheit von 2-Methyl-3-(3-tert-butylphenyl)-propanal (Formel II) bei einem Anteil von 0,2 bis 70 Gew.-% in dem Produktgemisch keine Beeinträchtigung der Riechstoffeigenschaften des 2-Methyl-3-(4-tert-butylphenyl)-propanals (Formel I) dar. Die Schrift C. Sell, Angew. Chem. 2006, 118, 6402-6410 beschreibt 2-Methyl-3-(3-tert-butylphenyl)-propanal (Formel II) als intensiver riechend als 2-Methyl-3-(4-tert-butylphenyl)-propanal (Formel I). Daher war bis vor kurzem ein gewisser Gehalt an 2-Methyl-3-(3-tert-butylphenyl)-propanal (Formel II) in Riechstoff-Kompositionen, die die vorgenannten Maiglöckchen-Riechstoffe enthalten, aus parfümistischen Aspekten sogar erwünscht.

Aufgrund geänderter Marktanforderungen war es jedoch notwendig, den Gehalt an Nebenkomponenten in 2-Methyl-3-(4-tert-butylphenyl)-propanal (Formel I), insbesonde-re 2-Methyl-3-(3-tert-butylphenyl)-propanal (Formel II), zu reduzieren, so dass in dem nach den oben beschriebenen Verfahren hergestellten 2-Methyl-3-(4-tert-butylphenyl)-propanal (Formel I) der Anteil an dem unerwünschten 2-Methyl-3-(3-tert-butylphenyl)-propanal (Formel II) mit üblicherweise mehr als 0,5 Gew.-% bezogen auf die Gesamtmasse der Verbindungen der Formeln I und II deutlich zu hoch liegt.

Aufgabe der vorliegenden Erfindung war daher die Bereitstellung eines einfachen und leicht implementierbaren Verfahrens, das 2-Methyl-3-(4-tert-butylphenyl)-propanal (Formel I) in einer solchen Isomerenreinheit zugänglich macht, dass der Gehalt von 2-Methyl-3-(3-tert-butylphenyl)-propanal (Formel II) in dem hergestellten 2-Methyl-3-(4-tert-butylphenyl)-propanal (Formel I) deutlich geringer ist als in den bisher kommerziell erhältlichen Produkten, ohne jedoch die Wirtschaftlichkeit des Verfahrens drastisch zu verringern.

Diese Aufgabe wird durch ein Verfahren zur Herstellung von 2-Methyl-3-(4-tert-butylphenyl)-propanal der Formel I 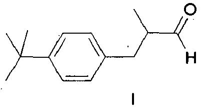 mit einen Gehalt von weniger als 0,3 Gew.-%, bevorzugt weniger als 0,1 Gew.-%, 2-Methyl-3-(3-tert-butylphenyl)-propanal der Formel II 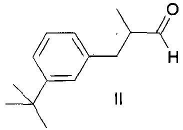 bezogen auf die Gesamtmasse der Verbindungen der Formeln I und II
ausgehend von 4-tert-Butyltoluol der Formel III 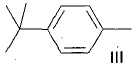 mit einem Gehalt von 0,5 bis 5 Gew.-% 3-tert-Butyltoluol der Formel IV 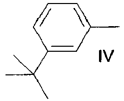 bezogen auf die Gesamtmasse der Verbindungen der Formeln III und IV als Ausgangsstoff
umfassend die Verfahrensschritte:
a) elektrochemische anodische Methoxylierung eines Gemisches enthaltend Verbindungen der Formeln III und IV sowie die als Zwischenprodukte anfallenden Benzylether der Formeln V und VI 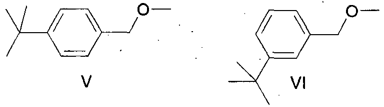 zu den Dimethylacetalen der Formeln VII und VIII 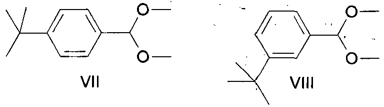 in einer Elektrolyselösung umfassend Methanol, mindestens ein Leitsalz sowie gegebenenfalls ein Cosolvens oder mehrere verschiedene Cosolventien,
   worin die Destillation in Verfahrensschritt b) zweistufig durchgeführt wird, indem in einer ersten Destillation mittels einer 1. Destillationskolonne ein Destillat am Kopf der 1. Destillationskolonne abdestilliert wird, welches zu mehr als 80 Gew.-% eine Mischung enthält, die aus nicht umgesetzten Ausgangsprodukten der Formeln III und IV, sowie den als Zwischenprodukten in Verfahrensschritt a) anfallenden Benzylethern der Formeln V und VI und dem Benzaldehyd der Formel X 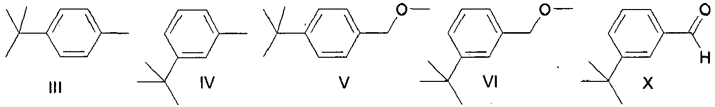 besteht,
   und in einer zweiten Destillation mittels einer 2. Destillationskolonne der Benzaldehyd der Formel IX 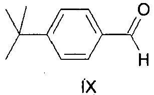 mit einem Gehalt von weniger als 0,5 Gew.-%, vorzugsweise weniger als 0,1 Gew.-% des Benzaldehyds der Formel X bezogen auf die Gesamtmasse der Verbindungen der Formeln IX und X aus dem Sumpf der ersten Destillation am Kopf der 2. Destillationskolonne abgetrennt wird, und wobei gegebenenfalls das am Kopf der 1. Destillationskolonne erhaltene Destillat, das die Verbindungen der Formeln III, IV, V, VI und X enthält, zusammen mit dem als Ausgangstoff eingesetzten 4-tert-Butyltoluol der Formel III wieder in Verfahrensschritt a) eingesetzt wird;
b) Hydrolyse des in Verfahrensschritt a) gebildeten Gemisches der Dimethylacetale der Formeln VII und VIII zu den entsprechenden Benzaldehyden der Formeln IX und X 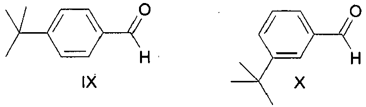 gefolgt von einer Abreicherung des Benzaldehyds der Formel X mittels Destillation;
c) Umsetzung des in Verfahrensschritt b) erhaltenen Gemisches der Benzaldehyde der Formeln IX und X, wobei das Gemisch weniger als 0,3 Gew.-% des Benzaldehydes der Formel X enthält, mit Propanal unter basischen Bedingungen zu den Dehydrozimtaldehyden der Formeln XI und XII; 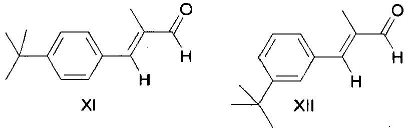
d) Katalytische Hydrierung der in Verfahrensschritt c) hergestellten Dehydrozimtaldehyde der Formeln XI und XII zu den Propanalen der Formeln I und II, 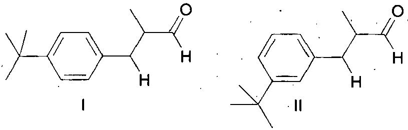 wobei der Gehalt des Propanals der Formel II weniger als 0,3 Gew.-% bezogen auf die Gesamtmasse der Verbindungen der Formeln I und II beträgt, gegebenenfalls gefolgt von einer Abtrennung von Lösungsmittelresten, Vorprodukten und Nebenprodukten mittels Destillation,
e) gegebenenfalls Reindestillation der in Verfahrensschritt d) gewonnenen Propanale der Formeln I und II zur Abreicherung des Propanals der Formel II,
gelöst.

In Verfahrensschritt a) des erfindungsgemäßen Verfahrens wird ein Gemisch enthaltend 4-tert-Butyltoluol der Formel III 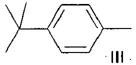 mit einem Gehalt von 0,5 bis 5 Gew.-% 3-tert-Butyltoluol der Formel IV 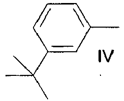 bezogen auf die Gesamtmasse der Verbindungen der Formeln III und IV als Ausgangsstoff, sowie die als Zwischenprodukte anfallenden Benzylether der Formeln V und VI 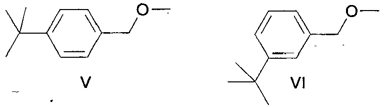 in einer Elektrolyselösung umfassend Methanol, mindestens ein Leitsalz sowie gegebenenfalls ein Cosolvens oder mehrere verschiedene Cosolventien durch elektrochemische anodische Methoxylierung zu den Dimethylacetalen der Formeln VII und VIII 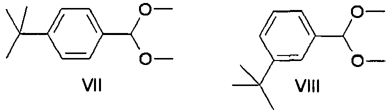 umgesetzt.

Die elektrochemische anodische Methoxylierung von Toluolderivaten ist prinzipiell bekannt und wird beispielsweise in EP 0 638 665 und der dort zitierten Literatur wie beispielsweise in DE-A 41 06 661 und EP 0 012 240, sowie in den Schriften: H. Pütter, (2001) Industrial electroorganic chemistry. In: H. Lund, O. Hammerich (Hrsg.) Organic electrochemistry, 4th ed., Marcel Dekker, New York, NY 2001, S. 1259-1307, sowie P. Loyson, S. Gouws, B. Zeelie, S. Afr. J. Chem., 2002, 55, 125-131, und P. Loyson, S. Gouws, B. Barton, M. Ackermann, S. Afr. J. Chem., 2004, 57, 53-56 und der darin zitierten Literatur beschrieben, wobei die genannten Dokumente sowie alle anderen in der vorliegenden Anmeldung zitierten Dokumente durch Verweis vollumfänglich in die vorliegende Offenbarung einbezogen sind (= incorporated by reference in their entirety).

Die Elektrolyselösung enthält im Rahmen des erfindungsgemäßen Verfahrens neben den Ausgangsstoffen der Formeln III und IV (Toluole) sowie den als Zwischenprodukten anfallenden Benzylethern der Formeln V und VI mindestens Methanol sowie mindestens ein Leitsalz.

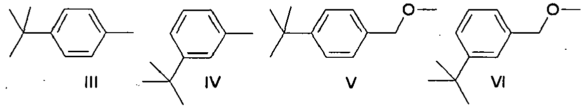

Bei den Leitsalzen, die in der Elektrolyselösung enthalten sein können, handelt es sich im Allgemeinen um Alkali-, Tetra(C₁- bis C₆-alkyl)ammonium-, bevorzugt Tri(C₁- bis C₆-alkyl)methylammoniumsalze. Als Gegenionen kommen Sulfat, Hydrogensulfat, Alkyl-sulfate, Arylsulfate, Alkylsulfonate, Arylsulfonate, Halogenide, Phosphate, Carbonate, Alkylphosphate, Alkylcarbonate, Nitrat, Alkoholate, Tetrafluorborat oder Perchlorat in Betracht.

Weiterhin kommen die von den vorstehend genannten Anionen abgeleiteten Säuren als Leitsalze in Betracht, also zum Beispiel Schwefelsäure, Sulfonsäuren sowie Carbonsäuren.

Daneben eignen sich als Leitsalze auch ionische Flüssigkeiten. Geeignete ionische Flüssigkeiten sind beschrieben in "Ionic Liquids in Synthesis", Hrsg. Peter Wasserscheid, Tom Welton, Verlag Wiley VCH, 2003, Kap. 1 bis 3 sowie in der DE-A 102004011427.

Die in Verfahrensschritt a) des erfindungsgemäßen Verfahrens bevorzugten Leitsalze sind Methyltributylammoniummethylsulfat (MTBS), Methyltriethylammoniummethylsulfat, Natriummethylsulfat, Natriumethansulfonat und Schwefelsäure.

Im Rahmen einer vorteilhaften Ausführungsform von Verfahrensschritt a) des erfindungsgemäßen Verfahrens wählt man die Konzentration des Leitsalzes in der Elektrolyselösung üblicherweise im Bereich von 0,1 bis 30 Gewichtsprozent (Gew.-%), bevorzugt im Bereich von 0,2 bis 10 Gew.-%, besonders bevorzugt von 0,25 bis 4 Gew.-%. Alle Gew.-% Angaben sind bezogen auf das Gesamtgewicht der Elektrolyselösung.

Eine weitere bevorzugte Ausführungsform von Verfahrensschritt a) des erfindungsgemäßen Verfahrens ist dadurch gekennzeichnet, dass man die elektrochemische anodische Methoxylierung bei einer Temperatur der Elektrolyselösung im Bereich von 35 bis 70°C, insbesondere im Bereich von 45 bis 60°C durchführt.

Darüber hinaus führt man den Verfahrensschritt a) des erfindungsgemäßen Verfahrens bevorzugt so durch, dass man die elektrochemische anodische Methoxylierung bei einem Absolutdruck im Bereich von 500 bis 100000 mbar, bevorzugt bei einem Absolutdruck im Bereich von 1000 bis 4000 mbar durchführt.

Gegebenenfalls setzt man der Elektrolyselösung übliche Cosolventien zu. Dabei han-delt es sich um die in der organischen Chemie allgemein üblichen inerten Lösungsmit-tel mit einem hohen Oxidationspotential. Beispielhaft genannt seien Dimethylcarbonat oder Propylencarbonat. Im Rahmen einer bevorzugten Ausführungsform führt man den Verfahrensschritt a) des erfindungsgemäßen Verfahrens daher in Gegenwart von Dimethylcarbonat und/oder Propylencarbonat als Cosolventien durch.

Als Cosolvens ist grundsätzlich auch Wasser geeignet, der Anteil von Wasser im Elektrolyten (=Elektrolyselösung) beträgt bevorzugt kleiner gleich 20 Gew.-%, insbesondere kleiner gleich 10 Gew.-%, vorzugsweise kleiner gleich 5 Gew.-%, insbesondere kleiner gleich 0,05 Gew.-%.

In einer weiteren bevorzugten Ausführungsform von Verfahrensschritt a) des erfindungsgemäßen Verfahren wird ein Elektrolyt (=Elektrolyselösung) eingesetzt, dessen Wassergehalt üblicherweise im Bereich von 0,001 bis 5 Gew.-%, bevorzugt im Bereich von 0,01 bis 1 Gew.-%, besonders bevorzugt im Bereich 0,01 bis 0,5 Gew.-% liegt. Alle Gew.-% Angaben sind bezogen auf das Gesamtgewicht der Elektrolyselösung zu Reaktionsbeginn.

Die vorliegenden Erfindung umfasst weiterhin die Erkenntnis, dass es vorteilhaft ist, wenn der Wassergehalt der Elektrolyselösung während des gesamten Verfahrensschritts a) kleiner gleich 20 Gew.-%, insbesondere kleiner gleich 10 Gew.-%, vorzugsweise kleiner gleich 5 Gew.-%, insbesondere kleiner gleich 0,05 Gew.-%, inbesondere kleiner gleich 0,01 Gew.-% beträgt.

Die Anteile an Wasser lassen sich durch verschiedene in der Literatur bekannte Methoden bestimmen. Besonders gut geeignet ist die Karl-Fischer-Titration. Das Wasser kann als Cosolvens dem Elektrolyten zugesetzt worden sein, einem der Einsatzstoffe wie beispielsweise Methanol oder Schwefelsäure entstammen oder durch die Rückführung von tert-Butylbenzaldehyd in die Synthese (siehe beispielsweise Schritt 2.3 aus Beispiel 2) eingetragen werden. Wird tert-Butylbenzaldehyd in die elektrochemische Methoxylierung (Verfahrensschritt a)) zurückgeführt, so erfolgt in dem Elektrolyten im sauren Milieu (pH < 7) die Acetalisierung des tert-Butylbenzaldehyds zum entsprechenden tert-Butylbenzaldehyddimethylacetal unter Freisetzung von einem Äquivalent Wasser. Vorzugsweise wird das bei der Acetaliserung von tert-Butylbenzaldehyd entstehende Wasser aus dem Gleichgewicht entfernt.

Die Entfernung des Wassers kann destillativ, vorzugsweise durch Verwendung eines Lösungsmittels und/oder Schleppmittels wie beispielsweise Toluol oder Methanol erreicht werden.

Eine andere Möglichkeit zur Wasserentfernung aus dem Elektrolyten besteht in der Verwendung von wasserentziehenden Mitteln wie beispielsweise Orthoestern.

In einer bevorzugten Ausführungsform der Erfindung enthält die Elektrolyselösung in Verfahrensschritt a) 0,1 bis 10 Gew.-% eines Orthoesters oder Mischungen verschiedener Orthoester. Die Gew.-% beziehen sich auf das Gesamtgewicht der Elektrolyselösung.

In einer bevorzugten Ausführungsform der vorliegenden Erfindung enthält die Elektrolyselösung in Verfahrensschritt a) neben Methanol, mindestens einem Leitsalz sowie gegebenenfalls einem Cosolvens oder mehrere verschiedene Cosolventien 0,1 bis 10 Gew.-% bezogen auf das Gesamtgewicht der Elektrolyselösung, bevorzugt 0,2 bis 5 Gew.-%, ganz besonders bevorzugt 0,5 bis 5 Gew.-% eines Orthoesters oder Mischungen verschiedener Orthoester.

Unter dem Begriff Orthoester werden Alkyl- und Arylester der frei nicht bekannten Orthocarbonsäuren, also Verbindungen der allgemeinen Formel R₁-C(OR₂)₃ verstanden, wobei R₁ für H, C1 bis C6 Alkylreste steht und R₂ unabhängig von einander für C1 bis C6 Alkylreste stehen.

Als Orthoester geeignet sind beispielsweise Orthoameisensäureester (Orthoformiate) (R₁=H) oder Orthoessigsäureestern (Orthoacetaten, R₁= CH₃), sowie Mischungen daraus.

Als Orthoameisensäureester geeignet sind beispielsweise Orthoameisensäuretrimethylester (R₁=H, R₂=CH₃) oder Orthoameisensäureäuretriethylester (R₁= H, R₂= C₂H₅)

Als Orthoessigsäureester geeignet sind beispielsweise Orthoessigsäuretrimethylester (R₁= CH₃, R₂= CH₃) oder Orthoessigsäuretriethylester (R₁= CH₃, R₂= C₂H₅)

Bevorzugt setzt man Orthoameisensäuretrimethylester (R₁=H, R₂=CH₃) oder Orthoessigsäuretrimethylester (R1₁= CH₃, R₂= CH₃) ein, ganz besonders bevorzugt Orthoameisensäuretrimethylester (R₁=H, R₂=CH₃).

Die dabei als Koppelprodukte entstehenden Essigsäuremethyl- und -ethylester sowie Ameisensäuremethyl- und -ethylester können anschließend in einem folgenden Verfahrensschritt leicht destillativ aus dem Reaktionsgemisch entfernt werden. Bevorzugt ist die Verwendung von Orthoameisensäuretrimethylester (Trimethylorthoformiat, Trimethoxymethan, CAS 14-73-5).

In einer bevorzugten Ausführungsform der Erfindung wird die Menge an Orthoester so gewählt, dass das molare Verhältnis von Orthoester zur Summe der in der Elektrolyselösung (durch Hinzufügen oder Rückführung) enthaltenden tert-Butylbenzaldehyde im Bereich von 0,1 bis 1,5 zu 1, vorzugsweise im Bereich von 0,1 bis 1 zu 1 liegt.

In einer Ausführungsform wird der Orthoester, beispielsweise Trimethylorthoformiat, portionsweise und/oder kontinuierlich zum Elektrolyten zudosiert.

Eine weitere Möglichkeit zur Wasserentfernung aus dem Elektrolyten besteht in der Verwendung von Ionentauschern oder Molekularsieben. Ionentauscher, auch Ionenaustauscher genannt, und Molekularsiebe sind als solche bekannt. Molekularsiebe werden vorzugsweise aus natürlichen und synthetischen Zeolithen gewählt, die in Form von Kugeln (Perlen), als Pulver oder Stäbchen vorliegen können. Bevorzugt setzt man Molekularsieb 4Å, besonders bevorzugt Molekularsieb 3Å ein.

In einer Ausführungsform der vorliegenden Erfindung versetzt man den Elektrolyten aus Verfahrensschritt a) mit Molekularsieb oder vorzugsweise Ionentauscher und lässt das Molekularsieb bzw. den Ionentauscher auf den Elektrolyten einwirken, beispielsweise indem man die Suspension von Molekularsieb bzw. Ionentauscher in dem Elektrolyten rührt, und zwar dauernd oder während gewisser Intervalle. Statt Rühren kann man auch Schütteln oder Umpumpen, bis ein Wassergehalt < 0,5 Gew.-% erreicht ist. Nach der Einwirkung von Molekularsieb oder Ionenaustauscher auf den Elektrolyten ist es erforderlich, das Molekularsieb bzw. den Ionentauscher abzutrennen. Das Abtrennen kann man durch Abdestillieren oder Abdekantieren des Elektrolyten oder bevorzugt durch Filtration bewerkstelligen.

In einer Ausführungsform der Erfindung wird die Elektrolyselösung vor der Durchführung von Verfahrensschritt a) über Molsieb, bevorzugt über Molekularsieb 4Å, besonders bevorzugt über Molekularsieb 3 Å bis zum einem Wassergehalt von kleiner gleich 0.05 Gew.% getrocknet und das Molsieb vor Verfahrensschritt a) durch Filtration vom Elektrolyten abgetrennt.

Die zur Wasserentfernung benutzten Molekularsiebe können nach der Abtrennung regeneriert (z.B. durch Durchströmen mit Heißluft oder erhitzen im Vakuum) und erneut eingesetzt werden. Solche Verfahren sind dem Fachmann bekannt.

Vorteilhaft an der Verwendung von Elektrolyten mit einem reduzierten Wassergehalt in der elektrochemischen Methoxylierung sind beispielsweise eine höhere Selektivität der Reaktion sowie ein geringerer spezifischer elektrischer Energieverbrauch bezogen auf das Wertprodukt (in kg) verglichen mit dem nicht oder nur teilweise getrockneten, ansonsten aber identischen Elektrolyten.

Der Verfahrensschritt a) des erfindungsgemäßen Verfahrens kann in allen üblichen geteilten oder ungeteilten Elektrolysezellentypen durchgeführt werden. Er kann mit gutem Erfolg sowohl diskontinuierlich als auch kontinuierlich durchgeführt werden. Im Rahmen einer bevorzugten Ausführungsform führt man den Verfahrensschritt a) des erfindungsgemäßen Verfahrens kontinuierlich durch. Vorzugsweise arbeitet man kontinuierlich mit ungeteilten Durchflusszellen.

Ganz besonders geeignet sind bipolar geschaltete Kapillarspaltzellen oder Plattenstapelzellen, bei denen die Elektroden als Platten ausgestaltet sind und planparallel angeordnet sind (Ullmann's Encyclopedia of Industrial Chemistry, 1999 electronic release, Sixth Edition, VCH-Verlag Weinheim, Volume Electrochemistry, Chapter 3.5 special cell designs sowie Chapter 5, Organic Electrochemistry, Subchapter 5.4.3.2 Cell Design). Als Elektrodenmaterial sind Edelmetalle wie Platin, Mischoxidelektroden wie RuOxTiOx (sogenannte DSA-Elektroden) oder kohlenstoffhaltige Materialien wie Graphit, Glassy Carbon oder Diamantelektroden bevorzugt. Ganz besonders bevorzugt werden Graphitelektroden eingesetzt. Im Rahmen einer bevorzugten Ausführungsform führt man den Verfahrensschritt a) des erfindungsgemäßen Verfahrens unter Verwendung einer Plattenstapelzelle durch.

Die Stromdichten, bei denen man den Verfahrensschritt a) durchführt, betragen im Allgemeinen 1 bis 1000 mA/cm², bevorzugt 10 bis 100 mA/cm². Besonders bevorzugt wird Verfahrensschritt a) des erfindungsgemäßen Verfahrens bei Stromdichten zwischen 10 und 50 mA/cm² durchgeführt. Im Allgemeinen wird bei Normaldruck gearbeitet. Höhere Drücke werden bevorzugt dann angewandt, wenn bei höheren Temperaturen gearbeitet werden soll, um ein Sieden der Ausgangsverbindungen bzw. des Lösungsmittels zu vermeiden.

Als Anodenmaterialien eignen sich beispielsweise Edelmetalle wie Platin oder Metalloxide wie Rutheniumoxid oder Chromoxid oder Mischoxide des Typs RuOₓ, TiOₓ sowie Diamantelektroden. Bevorzugt sind Graphit oder Kohleelektroden.

Als Kathodenmaterialien kommen beispielsweise Eisen, Stahl, Edelstahl, Nickel oder Edelmetalle wie Platin sowie Graphit oder Kohlematerialien sowie Diamantelektroden in Betracht. Bevorzugt ist das System Graphit als Anode und Kathode sowie Graphit als Anode und Nickel, Edelstahl oder Stahl als Kathode.

Bevorzugt wird ein erfindungsgemäßes Verfahren, worin das in Verfahrensschritt a) als Ausgangsstoff eingesetzte 4-tert-Butyltoluol der Formel III einen Gehalt von 2 bis 4 Gew.-%, besonders bevorzugt 2,5 bis 3,5 Gew.-% 3-tert-Butyltoluol der Formel IV bezogen auf die Gesamtmasse der Verbindungen der Formeln III und IV aufweist. Neben dem jeweils neu als Ausgangsstoff eingesetzten 4-tert-Butyltoluol können auch die in Verfahrensschritt b) nicht umgesetzten und zurück gewonnenen Ausgangsprodukte der Formeln III und IV sowie die als Zwischenprodukte gebildeten Benzylether der Formeln V und VI dem neuen, das heißt erstmalig in Verfahrensschritt a) eingesetzten 4-tert-Butyltoluol der Formel III mit einen Gehalt von 0,5 bis 5, bevorzugt 2 bis 4 Gew.-%, besonders bevorzugt 2,5 bis 3,5 Gew.-% 3-tert-Butyltoluol der Formel IV zugesetzt werden. Der Anteil des 3-tert-Butyltoluols der Formel IV in dem in Verfahrensschritt b) zurück gewonnenen 4-tert-Butyltoluols der Formel III weicht üblicherweise von dem in dem neu eingesetzten 4-tert-Butyltoluol vorhanden Anteil an 3-tert-Butyltoluol ab.

In Verfahrensschritt b) des erfindungsgemäßen Verfahrens erfolgt die Hydrolyse des in Verfahrensschritt a) gebildeten Gemisches der Dimethylacetale der Formeln VII und VIII zu den entsprechenden Benzaldehyden der Formeln IX und X 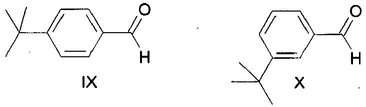 gefolgt von einer Abreicherung des Benzaldehyds der Formel X mittels Destillation.

Die Hydrolyse der Dimethylacetale der Formeln VII und VIII zu den entsprechenden Benzaldehyden der Formeln IX und X in Gegenwart von Wasser im Sauren ist prinzipiell bekannt und wird beispielsweise in H. Pütter, (2001) Industrial electroorganic chemistry. In: H. Lund, O. Hammerich (Hrsg.) Organic electrochemistry, 4th ed., Marcel Dekker, New York, NY 2001, S. 1288-1289, oder K. Schwetlick, Organikum, 21. Auflage 2001, Wiley-VCH, Weinheim, S. 182, beschrieben.

Der unerwünschte Benzaldehyld der Formel X wird mittels Destillation abgetrennt. Dabei wird bevorzugt ein Benzaldehyd der Formel IX erhalten, der einen Gehalt von weniger als 0,5 Gew.-%, bevorzugt weniger als 0,3 Gew.-%, bevorzugt weniger als 0,2 Gew.-%, insbesondere weniger als 0,1 Gew.-% des Benzaldehydes der Formel X bezogen auf die Gesamtmasse der Verbindungen der Formeln IX und X aufweist.

Bevorzugt wird ein erfindungsgemäßes Verfahren, worin das nach der Destillation in Verfahrensschritt b) erhaltene Gemisch der Benzaldehyde der Formeln IX und X einen Gehalt von weniger als 0,1 Gew.-% des Benzaldehydes der Formel X bezogen auf die Gesamtmasse der Verbindungen der Formeln IX und X aufweist.

Bevorzugt weist die in Verfahrensschritt b) eingesetzte 1. Destillationskolonne mehr als 40, insbesondere mehr als 50 theoretische Böden auf. Die Kolonnen sind vorzugsweise mit kommerziell erhältlichen Metall-Gewebepackungen ausgerüstet, wie sie von den Firmen Montz oder Sulzer erhältlich sind.

Die bevorzugte zweistufige Destillation kann prinzipiell auch in einer einzigen Trennwandkolonne gleichzeitig ausgeführt werden. Bevorzugt wird jedoch eine Destillation, in der die beiden Destillationsschritte getrennt voneinander, besonders bevorzugt in zwei verschiedenen, jeweils an die speziellen Trennungsanforderungen angepassten Destillationskolonnen ausgeführt werden.

In Verfahrensschritt c) wird das in Verfahrensschritt b) erhaltene Gemisch der Benzaldehyde der Formeln IX und X, wobei das Gemisch weniger als 0,3 Gew.-% des Benzaldehydes der Formel X enthält, mit Propanal unter basischen Bedingungen zu den Dehydrozimtaldehyden der Formeln XI und XII 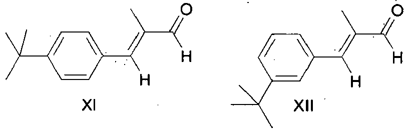 umgesetzt.

In Verfahrensschritt d) werden die in Verfahrensschritt c) hergestellten Dehydrozimtaldehyde der Formeln XI und XII zu den Propanalen der Formeln I und II katalytisch hydriert, 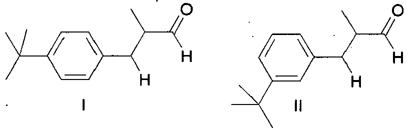 wobei der Gehalt des Propanals der Formel II weniger als 0,3 Gew.-% bezogen auf die Gesamtmasse der Verbindungen der Formeln I und II beträgt, gegebenenfalls gefolgt von einer Abtrennung von Lösungsmittelresten, Vorprodukten und Nebenprodukten mittels Destillation.

In Verfahrensschritt e) werden gegebenenfalls die in Verfahrensschritt d) gewonnenen Propanale der Formeln I und II zur Abreicherung des Propanals der Formel II einer Reindestillation unterworfen.

Die Verfahrensschritte c) und d) werden üblicherweise wie in WO 2001/027061 oder in H. Surburg, J. Panten, Common Fragrance and Flavor Materials, 5. Auflage, Wiley-VCH, Weinheim 2006, S. 115-117 beschrieben durchgeführt.

Bevorzugt wird ein erfindungsgemäßes Verfahren, worin in Verfahrensschritt d) das Rohprodukt aus der katalytischen Hydrierung zur Abtrennung von Lösungsmittelresten, Vorprodukten und Nebenprodukten einer Destillation unterworfen wird.

Falls das erhaltene Propanal der Formel I noch einen zu hohen Gehalt an dem Propanal der Formel II aufweist, kann der Gehalt an Propanal der Formel II durch eine Reindestillation weiter abgereichert werden. Eine solche Reindestillation wird bevorzugt in einer Trennwandkolonnne durchgeführt, wie sie beispielsweise in DE10223970 beschrieben wird.

Besonders bevorzugt wird ein erfindungsgemäßes Verfahren, worin das nach der Destillation in Verfahrensschritt d) oder e) erhaltene Gemisch der Propanale der Formeln I und II einen Gehalt von weniger als 0,05 Gew.-% des Propanals der Formel II bezogen auf die Gesamtmasse der Verbindungen der Formeln I und II aufweist

Ein weiterer Gegenstand der Erfindung ist auch ein Verfahren zur Herstellung von 4-tert-Butylbenzaldehyd der Formel IX 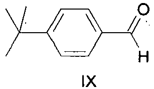 mit einen Gehalt von weniger als 0,1 Gew.-% 3-tert-Butylbenzaldehyd der Formel X 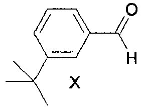 bezogen auf die Gesamtmasse der Verbindungen der Formeln IX und X,
ausgehend von 4-tert-Butyltoluol der Formel III 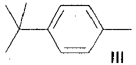 mit einem Gehalt von 0,5 bis 5 Gew.-% 3-tert-Butyltoluol der Formel IV 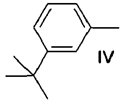 bezogen auf die Gesamtmasse der Verbindungen der Formeln III und IV als Ausgangsstoff
umfassend die Verfahrensschritte:
a) elektrochemische anodische Methoxylierung eines Gemisches enthaltend Verbindungen der Formeln III und IV sowie die als Zwischenprodukte anfallenden Benzylether der Formeln V und VI 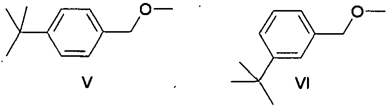 zu den Dimethylacetalen der Formeln VII und VIII 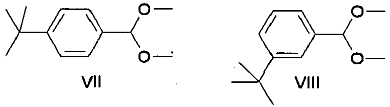 in einer Elektrolyselösung umfassend Methanol, mindestens ein Leitsalz sowie gegebenenfalls ein Cosolvens oder mehrere verschiedene Cosolventien;
b) Hydrolyse des in Verfahrensschritt a) gebildeten Gemisches der Dimethylacetale der Formeln VII und VIII zu den entsprechenden Benzaldehyden der Formeln IX und X 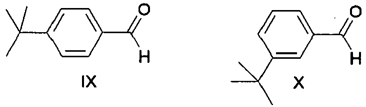 gefolgt von einer Abreicherung des Benzaldehyds der Formel X mittels Destillation,
   wobei die Destillation in Verfahrensschritt b) zweistufig durchgeführt wird,
   indem in einer ersten Destillation mittels einer 1. Destillationskolonne ein Destillat am Kopf der 1. Destillationskolonne abdestilliert wird, welches zu mehr als 80 Gew.-% eine Mischung enthält, die aus nicht umgesetzten Ausgangsprodukten der Formeln III und IV, sowie den als Zwischenprodukten in Verfahrensschritt a) anfallenden Benzylethern der Formeln V und VI und dem Benzaldehyd der Formel X 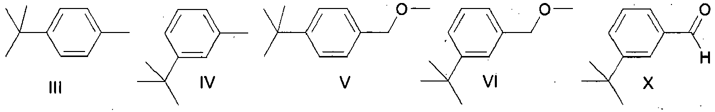 besteht,
   und in einer zweiten Destillation mittels einer 2. Destillationskolonne der Benzaldehyd der Formel IX 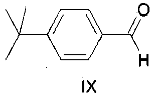 mit einem Gehalt von weniger als 0,5 Gew.-%, vorzugsweise weniger als 0,1 Gew.-% des Benzaldehyds der Formel X bezogen auf die Gesamtmasse der Verbindungen der Formeln IX und X aus dem Sumpf der ersten Destillation am Kopf der 2. Destillationskolonne abgetrennt wird, und wobei gegebenenfalls das am Kopf der 1. Destillationskolonne erhaltene Destillat, das die Verbindungen der Formeln III, IV, V, VI und X enthält, zusammen mit dem als Ausgangstoff eingesetzten 4-tert-Butyltoluol der Formel III wieder in Verfahrensschritt a) eingesetzt wird.

Bevorzugte Ausführungsformen des Verfahrensschrittes a) wurden bereits vorangehend beschrieben.

Während der Sumpf der zweiten Destillation in Verfahrensschritt b) üblicherweise verworfen wird, kann man das am Kopf der 1. Destillationskolonne erhaltene Destillat wieder teilweise oder vollständig dem Gemisch zugeben, das in Verfahrensschritt a) der elektrochemischen, anodischen Methoxylierung unterworfen wird.

Bevorzugt ist ein Verfahren zur Herstellung von 4-tert-Butylbenzaldehyd der Formel IX, wobei das in Verfahrensschritt b) am Kopf der 1. Destillationskolonne erhaltene Destillat zusammen mit dem als Ausgangstoff eingesetzten 4-tert-Butyltoluol der Formel III wieder in Verfahrensschritt a) eingesetzt wird.

Bei der wiederholten Wiederverwendung des in Verfahrensschritt b) am Kopf der 1. Destillationskolonne erhaltenen Destillates in Verfahrensschritt a) reichert sich insbesondere der unerwünschte Benzaldehyd der Formel X und weiterhin der Benzylether der Formel VI an.

Die Zusammensetzung des am Kopf der 1. Destillationskolonne erhaltenen Destillates kann beispielsweise mittels Gaschromatographie bestimmt werden.

Bevorzugt wird nach wiederholter Durchführung der Verfahrensschritte a) und b) ein Anteil von bis zu 50 Gew.-%, besonders bevorzugt zwischen 1 und 20 Gew.-%, insbesondere zwischen 2 und 7 Gew.-% des am Kopf der 1. Destillationskolonne erhaltenen Destillates verworfen.

Besonders bevorzugt besteht der verworfene Anteil des Destillates zu mehr als 15 Gew.-%, insbesondere zu mehr als 20 Gew.-% aus dem Benzylether der Formel VI und dem Benzaldehyd der Formel X bezogen auf die Masse der Fraktion.

Alternativ kann der Benzaldehyd der Formel X aus dem Destillat, das am Kopf der 1. Destillationskolonne erhaltenen wird, auch durch eine chemische Umwandlung in einen neuen Stoff, insbesondere einen Feststoff, der sich in seinen physikalischen Eigenschaften von den übrigen flüssige Bestandteilen der Formeln III, IV, V und VI deutlich unterscheidet, entfernt werden.

Weiterhin bevorzugt ist daher ein Verfahren zur Herstellung von 4-tert-Butylbenzaldehyd der Formel IX, wobei der Benzaldehyd der Formel X aus dem Destillat, das am Kopf der 1. Destillationskolonne erhaltenen wird, nach chemischer Umwandlung in einen Feststoff von den weiteren flüssigen Bestandteilen des Destillates, insbesondere den Verbindungen der Formeln III, IV, V und VI, abgetrennt wird, und anschließend das weitgehend Aldehyd-freie aufgereinigte Destillat zusammen mit dem als Ausgangstoff eingesetzten 4-tert-Butyltoluol der Formel III wieder in Verfahrensschritt a) eingesetzt wird.

Dem Fachmann sind aus den klassischen Methoden zur Charakterisierung und Reinigung von flüssigen Aldehyden durch Umwandlung der Aldehyd-Funktion verschiedene Derivate bekannt, die entweder bei Raumtemperatur fest sind oder die ein anderes Löslichkeitsprofil als die flüssigen Bestandteile der Formeln III, IV, V und VI besitzen. Der Benzaldehyd der Formel X kann beispielsweise in ein bei Raumtemperatur festes Hydrazonderivat, Semicarbazonderivat, Oxim, Imin oder Bisulfit-Addukt umwandelt werden und durch bekannte Fest-Flüssig-Trennmethoden wie beispielsweise Filtration oder Zentrifugieren, abgetrennt werden. Im Falle des Bisulfit-Adduktes kann dieses auch durch Auflösen in Wasser von den übrigen hydrophoben flüssigen Bestandteilen der Formeln III, IV, V und VI abgetrennt werden.

Besonders bevorzugt ist somit das vorangehend beschriebene Verfahren zur Herstellung von 4-tert-Butylbenzaldehyd der Formel IX, worin der Benzaldehyd der Formel X aus dem Destillat, das am Kopf der 1. Destillationskolonne erhaltenen wird, durch chemische Umwandlung zu einem bei Raumtemperatur festen Hydrazonderivat, Semicarbazonderivat, Oxim, Imin oder Bisulfit-Addukt von den weiteren flüssigen Bestandteilen der Formeln III, IV, V und VI entfernt wird, und anschließend das weitgehend Aldehyd-freie aufgereinigte Destillat zusammen mit dem als Ausgangstoff eingesetzten 4-tert-Butyltoluol der Formel III wieder in Verfahrensschritt a) eingesetzt wird.

Der nach dem vorangehend beschriebenen Verfahren zur Herstellung von 4-tert-Butylbenzaldehyd der Formel IX erhältliche Benzaldehyd der Formel IX, der einen Gehalt von weniger als 0,1 Gew.-% des Benzaldehyds der Formel X bezogen auf die Gesamtmasse der Verbindungen der Formeln IX und X aufweist, kann vorzugsweise in dem Verfahrensschritt c) des eingangs beschriebenen Verfahrens zur Herstellung von 2-Methyl-3-(4-tert-butylphenyl)-propanal der Formel I eingesetzt werden. Somit stellen die in dem vorangehend beschriebenen Verfahren zur Herstellung von 4-tert-Butylbenzaldehyd der Formel IX bevorzugten Ausführungsformen auch in den Verfahrensschritten a) und b) des Verfahrens zur Herstellung von 2-Methyl-3-(4-tert-butylphenyl)-propanal der Formel I besonders bevorzugte Ausführungsformen dar.

3-tert-Butylbenzaldehyd der Formel X ist als Intermediat bei der Herstellung von 2-Methyl-3-(4-tert-butylphenyl)-propanal unerwünscht.
3-tert-Butylbenzaldehyd der Formel X stellt jedoch einen wertvollen Ausgangstoff für andere Synthesen dar, da das 1,3-Substitutionsmuster nur schwer zugänglich ist. Seine Abtrennung, Aufreinigung und anderweitige Verwendung oder Vermarktung erhöht somit die Wirtschaftlichkeit des vorliegenden Gesamtprozesses.

Durch die Rückspaltung des Feststoffes, der nach Abtrennung von den weiteren flüssigen Bestandteilen des Destillates erhalten werden kann, ist es möglich, den Aldehyd der Formel X in einer Reinheit von mindestens 80 Gew.-% zu erhalten.

Ein weiterer Gegenstand der vorliegenden Erfindung ist daher auch ein Verfahren zur Herstellung des Benzaldehyds der Formel X 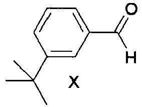 in einer Reinheit von mindestens 80 Gew.-% umfassend die Verfahrensschritte:
i) Chemische Umwandlung des Benzaldehyds der Formel X, der in dem Destillat enthalten ist, das am Kopf der 1. Destillationskolonne wie vorangehend beschrieben erhalten wird, in einen Feststoff;
ii) Abtrennung des in Verfahrensschritt i) gebildeten Feststoffes von den übrigen flüssigen Bestandteilen des Destillates, insbesondere den Verbindungen der Formeln III, IV, V und VI;
iii) Freisetzung des Benzaldehyds der Formel X aus dem in Verfahrensschritt ii) abgetrennten Feststoff.

Bevorzugt ist ein Verfahren zur Herstellung des Benzaldehyds der Formel X wie vorangehend beschrieben, wobei in Verfahrensschritt i) der Benzaldehyd der Formel X zu einem bei Raumtemperatur festen Hydrazonderivat, Semicarbazonderivat, Oxim, Imin oder Bisulfit-Addukt umgesetzt wird, in Verfahrensschritt ii) das in Verfahrensschritt i) gebildete Hydrazonderivat, Semicarbazonderivat, Oxim, Imin oder Bisulfit-Addukt von den übrigen flüssigen Bestandteilen des Destillates, insbesondere den Verbindungen der Formeln III, IV, V und VI abgetrennt wird, und in Verfahrensschritt iii) aus dem in Verfahrensschritt ii) abgetrennten Hydrazonderivat, Semicarbazonderivat, Oxim, Imin oder Bisulfit-Addukt der Benzaldehyd der Formel X freigesetzt wird.

Im Fall der Abtrennung des Benzaldehyds der Formel X aus dem Destillat, das am Kopf der 1. Destillationskolonne wie vorangehend beschrieben erhalten wird, in Form eines Hydrazonderivates, eines Semicarbazonderivates, des Oxim, eines Imins oder des Bisulfit-Addukt kann auch direkt mit einem dieser Derivate weitergearbeitet werden, ohne den 3-tert-Butylbenzaldehyd der Formel X erneut daraus freizusetzen. Ist das Syntheseziel z.B. 3-tert-Butylbenzylamin, wird der 3-tert-Butylbenzaldehyd der Formel X von den weiteren Bestandteilen des Destillates der 1. Destillationskolonne der Formeln III, IV, V und VI als 3-tert-Butylbenzaldehyd-Imin entfernt und das 3-tert-Butylbenzaldehyd-Imin nach gängigen Verfahren zum 3-tert-Butylbenzylamin reduziert.

Das erfindungsgemäße Verfahren zur Herstellung von 2-Methyl-3-(4-tert-butylphenyl)-propanal der Formel I zeichnet sich insbesondere durch seine einfache Integrierbarkeit in einen bestehenden Prozess aus, ohne die Rohstoffbasis, das heißt die Reinheit des Ausgangsstoffs 4-tert.-Butyltoluol der Formel III, oder die prinzipielle Syntheseroute ändern zu müssen. Auch das erfindungsgemäße Verfahren zur Herstellung von 4-tert-Butylbenzaldehyd der Formel IX zeichnet sich wie vorangehend beschrieben durch seine einfache Integrierbarkeit in einen bestehenden Prozess aus, ohne die Rohstoffbasis oder die prinzipielle Syntheseroute ändern zu müssen.

Die in Verfahrensschritt b) des erfindungsgemäßen Verfahrens zur Herstellung von 2-Methyl-3-(4-tert-butylphenyl)-propanal der Formel I und des erfindungsgemäßen Ver-fahrens zur Herstellung von 4-tert-Butylbenzaldehyd der Formel IX durchgeführte Destillation, insbesondere die zweistufige Destillation, die zur Abreicherung des 3-tert-Butylaldehyds der Formel X bei der Herstellung von 4-tert-Butylbenzaldehyd der Formel IX in hoher Reinheit dient, eröffnet einen wirtschaftlichen Zugang zu hochreinem 2-Methyl-3-(4-tert-butylphenyl)-propanal der Formel I verbunden mit der Möglichkeit zur Gewinnung des ansonsten nicht leicht zugänglichen 3-tert-Butylaldehyds der Formel X.

Die Erfindung wird durch folgende, die Erfindung jedoch nicht einschränkende Beispiele erläutert.

### Vergleichsbeispiel 1- Herstellung einer Mischung enthaltend 98,5 Gew.-% 2-Methyl-3-(4-tert-butylphenyl)-propanal und 0,7 bis 1,5 Gew.-% 2-Methyl-3-(3-tert-butylphenyl)-propanal

### Schritt 1.1.- Synthese der tert-Butylbenzaldehyd-Dimethylacetale (VII) und (VIII):

Eine Mischung bestehend aus 22 Gew.-% tert-Butyltoluol mit einem Gehalt von 96 Gew.-% 4-tert-Butyltoluol (III) und 3 Gew.-% 3-tert-Butyltoluol (IV), 77,5 Gew.-% Methanol als Lösungsmittel und 0,5 Gew.-% Schwefelsäure als Leitsalz wurde in einem kontinuierlich betriebenen Reaktor in der Kapillarspaltzelle an Grafitelektroden bei einer Temperatur von 45°C bis 60°C und einer Stromdichte von 42 A/dm² elektrolysiert, bis sich in der Reaktionsmischung ein Gehalt von etwa 14 Gew.-% 4-tert-Butylbenzaldehyd-Dimethylacetal (VII) einstellte.

Bei der wiederholten Durchführung von Schritt 1.1 wurde die Elektrolyse bei Temperaturen von 45°C bis 60°C durchgeführt, wobei in der Reaktionsmischung ein Gehalt von 12 bis 16 Gew.-% 4-tert-Butylbenzaldehyd-Dimethylacetal (VII) einstellt wurde.

### Schritt 1.2. - Hydrolyse der tert-Butylbenzaldehyd-Dimethylacetale (VII) und (VIII) zu den tert-Butylbenzaldehyden (IX) und (X) und destillative Aufreinigung:

Zur Aufarbeitung wurde kontinuierlich Reaktionsmischung aus der Elektrolysezelle ausgetragen und durch frische Reaktionsmischung bestehend aus 22 Gew.-% tert-Butyltoluol mit einem Gehalt von 96 Gew.-% 4-tert-Butyltoluol und 3 Gew.-% 3-tert-Butyltoluol, 77,5 Gew.-% Methanol als Lösungsmittel und 0,5 Gew.-% Schwefelsäure als Leitsalz ergänzt. Das Lösungsmittel Methanol wurde abdestilliert und das zurückbleibende Rohacetal mit Wasser hydrolysiert. Nach der Phasentrennung wurde ein Rohprodukt erhalten, das neben 4- und 3-tert-Butylbenzaldehyd noch nicht umgesetztes 3- und 4-tert-Butyltoluol sowie 3- und 4-tert-Butylbenzylmethylether als rückführbares Zwischenprodukte enthielt. Die destillative Aufarbeitung wurde zweistufig (Destillationskolonne 1, Destillationskolonne 2) durchgeführt. Als Destillationskolonne 1 diente eine gut wirksame Kolonne mit ca. 60 theoretischen Böden.

Zunächst wurden die leichter siedenden 3- und 4-tert-Butyltoluole sowie 3- und 4-tert-Butylbenzylmethylether (V und VI) am Kopf der Destillationskolonne 1 abgetrennt (Temperaturprofil: T_{Sumpf} = 158,5°C, T_{Kopf} = 142°C bei 35 mbar) und in die Elektrolyse zurückgegeben (siehe Schritt 1.3.).

Der Sumpf der Destillationskolonne 1, welcher 3-tert-Butylbenzaldehyd (X), 4-tert-Butylbenzaldehyd (IX) sowie Hochsieder enthielt, wurde in die Destillationskolonne 2 gegeben. In der Destillationskolonne 2 wurden die 4- und 3-tert-Butylbenzaldehyde am Kopf von den hochsiedenden Verunreinigungen abgetrennt und der Kondensation mit Propanal (siehe Schritt 1.4.) zugeführt. Der Destillationssumpf der Destillationskolonne 2 wurde verworfen.

Die Destillation wurde so durchgeführt, dass ein tert-Butylbenzaldehyd erhalten wurde, der zu 97 Gew.-% aus 4-tert-Butylbenzaldehyd (IX) und 1,5 Gew.-% 3-tert-Butylbenzaldehyd (X) bestand.

### Schritt 1.3. - Rückführung des Kopfproduktes der Destillationskolonne 1:

Das an der Destillationskolonne 1 anfallende Kopfprodukt wurde in die Synthese (Schritt 1.1.) zurückgeführt.

### Schritt 1.4. - Folgestufen:

Die folgenden Schritte wurden wie in der Schrift WO 2001/027061 beschrieben durchgeführt. Es wurde ein 2-Methyl-3-(tert-butylphenyl)-propanal erhalten, das etwa 98,5 Gew.-% 2-Methyl-3-(4-tert-butylphenyl)-propanal und etwa 1,1 Gew.-% 2-Methyl-3-(3-tert-butylphenyl)-propanal enthielt.

### Bei der wiederholten Durchführung der in Vergleichsbeispiel 1 beschriebenen Synthese wurden Gemische erhalten, die jeweils etwa 98,5 Gew.-% 2-Methyl-3-(4-tert-butylphenyl)-propanal und zwischen 0,7 bis 1,5 Gew.-% 2-Methyl-3-(3-tert-butylphenyl)-propanal enthielten.

### Beispiel 2 - Herstellung von 4-tert-Butylbenzaldehyd mit hoher Isomerenreinheit

### Schritt 2.1. - Synthese der tert-Butylbenzaldehyd-Dimethylacetale (VII) und (VIII):

Die Synthese wurde analog wie im Vergleichsbeispiel 1, Schritt 1.1 durchgeführt. Es wurden dieselben Einsatzstoffe verwendet.

### Schritt 2.2. - Hydrolyse der tert-Butylbenzaldehyd-Dimethylacetale (VII) und (VIII) zu den tert-Butylbenzaldehyden (IX) und (X) und destillative Aufreinigung:

Zur Aufarbeitung wurde kontinuierlich Reaktionsmischung aus der Elektrolysezelle ausgetragen und durch frische Reaktionsmischung ergänzt. Das Lösungsmittel wurde abdestilliert und das zurückbleibende Rohacetal mit Wasser hydrolysiert. Nach der Phasentrennung wurde ein Rohprodukt erhalten, das neben 4- und 3-tert-Butylbenz-aldehyd noch nicht umgesetztes 3- und 4-tert-Butyltoluol sowie 3- und 4-tert-Butylbenzylmethylether als rückführbares Zwischenprodukte enthielt. Die destillative Aufarbeitung wurde zweistufig (Destillationskolonne 1, Destillationskolonne 2) durchgeführt. Die Destillationskolonne 1 ist eine gut wirksame Kolonne mit ca. 60 theoretischen Böden.

Zunächst wurden die leichter siedenden 3- und 4-tert-Butyltoluole, 3- und 4-tert-Butylbenzylmethylether sowie 3-tert-Butylbenzaldehyd am Kopf der Destillationskolonne 1 abgetrennt (Temperaturprofil: T_{Sumpf} = 159°C, T_{Kopf} = 147°C bei 35 mbar) und in die Elektrolyse zurückgegeben (siehe Schritt 2.3.), wobei 4-tert-Butylbenzäldehyd im Sumpf der Destillationskolonne 1 anfiel.

Der Sumpf der Destillationskolonne 1, der insbesondere 4-tert-Butylbenzaldehyd sowie Hochsieder enthielt, wurde in die Destillationskolonne 2 gegeben. In der Destillationskolonne 2 wurde 4-tert-Butylbenzaldehyd (IX) am Kopf von den hochsiedenden Verunreinigungen abgetrennt und der Kondensation mit Propanal (siehe Schritt 2.4.) zugeführt. Der Destillationssumpf der Destillationskolonne 2 wurde verworfen.

Die Destillation wurde so durchgeführt, dass ein tert-Butylbenzaldehyd erhalten wurde, der zu 98-99 Gew.-% aus 4-tert-Butylbenzaldehyd (IX) und zu 0,02 - 0,08 Gew.-% aus 3-tert-Butylbenzaldehyd (X) bestand.

### Schritt 2.3. - Rückführung des Kopfproduktes der Destillationskolonne 1:

Das an der Destillationskolonne 1 anfallende Kopfprodukt wurde in die Synthese (Schritt 2.1. zurückgeführt). Da nach wiederholter Durchführung der Schritte 2.1 und 2.2 das zurückgeführte Kopfprodukt der Destillationskolonne 1 zunehmende Mengen an 3-tert-Butylbenzaldehyd (X) enthält und 3-tert-Butylbenzaldehyd destillativ nicht vom 4-tert-Butylbenzylmethylether (V) abgetrennt werden kann, wurde ein geringfügiger Teil (2-7 Gew%) des Kopfproduktes der Destillationskolonne 1 abgetrennt und verworfen.

### Schritt 2.4. - Folgestufen:

Die folgenden Schritte wurden wie in der Schrift WO 2001/027061 beschrieben durchgeführt. Es wurde ein 2-Methyl-3-(tert-butylphenyl)-propanal erhalten, dass zu > 99,0 Gew.-% aus 2-Methyl-3-(4-tert-butylphenyl)-propanal und < 0,05 Gew.-% 2-Methyl-3-(3-tert-butylphenyl)-propanal bestand.

### Beispiel 3 - Herstellung von 4-tert-Butylbenzaldehyd mit hoher Isomerenreinheit mit dem Leitsalz Methyltributylammoniummethylsulfat (MTBS)

Schritt 3.1. - Synthese der tert-Butylbenzaldehyd-Dimethylacetale (VII) und (VIII): Eine Mischung bestehend aus 23 Gew.-% tert-Butyltoluol mit einem Gehalt von 96 Gew.-% 4-tert-Butyltoluol (III) und 3 Gew.-% 3-tert-Butyltoluol (IV), 74 Gew.-% Methanol als Lösungsmittel und 3,0 Gew.-% Methyltributylammoniummethylsulfat (MTBS) als Leitsalz wurde in einem kontinuierlich betriebenen Reaktor in der Kapillarspaltzelle an Grafitelektroden bei einer Temperatur von 45°C bis 60°C und einer Stromdichte von 22 A/dm² elektrolysiert, bis sich in der Reaktionsmischung ein Gehalt von etwa 14 Gew.-% 4-tert-Butylbenzaldehyd-Dimethylacetal (VII) einstellte.

Bei der wiederholten Durchführung von Schritt 3.1 wurde die Elektrolyse bei Temperaturen von 45°C bis 60°C durchgeführt, wobei in der Reaktionsmischung ein Gehalt von 12 bis 16 Gew.-% 4-tert-Butylbenzaldehyd-Dimethylacetal (VII) einstellt wurde.

### Schritt 3.2. - Hydrolyse der tert-Butylbenzaldehyd-Dimethylacetale (VII) und (VIII) zu den tert-Butylbenzaldehyden (IX) und (X) und destillative Aufreinigung:

Zur Aufarbeitung wurde kontinuierlich Reaktionsmischung aus der Elektrolysezelle ausgetragen und durch frische Reaktionsmischung ergänzt. Das Lösungsmittel wurde abdestilliert und zur Abtrennung des Leitsalzes MTBS wurde eine Totalverdampfung im Vakuum in einem Dünschichtverdampfer bei 180°C bis 220°C und 10 mbar bis 20 mbar durchgeführt. Das nichtverdampfbare MTBS wurde in die Elektrolyse (Schritt 3.1.) zurückgegeben. Das Rohacetal wurde kondensiert und mit Wasser hydrolysiert. Nach der Phasentrennung wurde ein Rohprodukt erhalten, das neben 4- und 3-tert-Butylbenzaldehyd noch nicht umgesetztes 3- und 4-tert-Butyltoluol sowie 3- und 4-tert-Butylbenzylmethylether als rückführbares Zwischenprodukte enthielt. Die destillative Aufarbeitung wurde zweistufig (Destillationskolonne 1, Destillationskolonne 2) wie im Schritt 2.2. durchgeführt.

### Schritt 3.3. - Rückführung des Kopfproduktes der Destillationskolonne 1 Durchführung wie Schritt 2.3..

### Schritt 3.4. - Folgestufen:

Durchführung wie Schritt 2.4..

Diskontinuierliche Elektrolyse zur Herstellung von 4-tert-Butylbenzaldehyddimethyl-acetal (VII)
- Apparatur:: Ungeteilte Durchflusszelle mit 11 kreisförmigen Grafitelektroden (SGL MKUS F04, Abstand: 1 mm, 10 Spalte), Zellinhalt etwa 3 kg Elektrolyt
- Elektrolyt:: 600 g (20 Gew-%) Kopfprodukt der Destillationskolonne 1 (siehe Schritt 2.3 aus Beispiel 2 (enthält bis zu 20,1 Gew-% 3-tert-Butylbenzaldehyd), 2390 g (79.6 Gew-%) MeOH, 10,5 g konzentrierte Schwefelsäure (0,35 Gew-%, Gehalt >96%) sowie ein variabler Anteil Trimethylorthoformiat (sofern nicht anders angegeben)
- Elektrolyse: bis zum Vollumsatz von 4-tert-Butyltoluol (III), bestimmt durch Gaschromatographie
- Stromdichte:: 3.4 A dm-2 (sofern nicht anders angegeben).
- Temperatur:: 53°C (sofern nicht anders angegeben).

Das verwendete Kopfprodukt der Destillationskolonne 1 hat die in Tabelle B-1 gezeigte Zusammensetzung der Hauptkomponenten (Angabe in GC-FI-%, 600 g Einwaage). Bei den zu 100 % fehlenden GC-FI-% handelt es sich um nicht näher bestimmte Nebenkomponenten. Sofern Trimethylorthoformiat (TMOF) dem Elektrolyten zugesetzt wurde, wurde der Anteil von Methanol im Elektrolyten um den gleichen Wert reduziert, d.h. die Summe aus Methanol- und TMOF-Anteil im Elektrolyten ist konstant. Die angegebenen GC-FI-% werden für die Bilanzierung der Versuche GC-Gew-% gleichgesetzt, so dass sich die Gewichtsanteile der Hauptkomponenten zu den in Tabelle B-1 genannten Werten berechnen. Als Endpunkt der Elektrolyse wurde der Vollumsatz von 4-tert-Butyltoluol (III) gewählt und mittels Gaschromatographie bestimmt. Die angegebene elektrische Ladungsmenge bis zum Vollumsatz von III wird berechnet aus der Elektrolysedauer multipliziert mit der Stromstärke und der Anzahl der Elektrolysespalte der verwendeten Kapillarspaltzelle.

Nach Beendigung der Elektrolyse wurden die Elektrolyseausträge destillativ vom Me-OH befreit und der Rückstand zur Bestimmung der Ausbeuten destilliert. In Tabelle B-1 sind die so gefundenen Gewichte der Hauptkomponenten angegeben, berechnet aus den Gaschromatogrammen der einzelnen Fraktionen der Destillation.

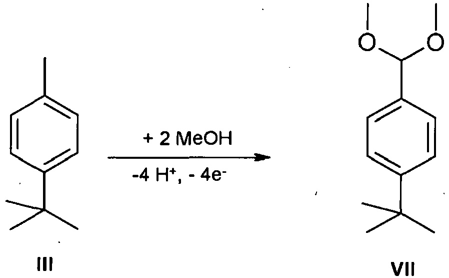

Beispiel 4 beschreibt den Referenzversuch in dieser Versuchsreihe ohne den Zusatz von Trimethylorthoformiat (TMOF) zum Elektrolyten. Vollumsatz von III wird nach 495 Ah erreicht und liefert 11,2 g 4-tert-Butylbenzaldehyd (IX) sowie 254,0 g 4-tert-Butylbenzaldehyddimethylacetal (VII), das durch Hydrolyse nach oben bereits beschriebenen Verfahren in 4-tert-Butylbenzaldehyd (IX) überführt werden kann.

Beispiel 5 beschreibt den Einfluss von 1 Gew-% TMOF im Elektrolyten. Vollumsatz von III wird nach 387 Ah erreicht und liefert 6,9 g 4-tert-Butylbenzaldehyd (IX) sowie 287 g 4-tert-Butylbenzaldehyddimethylacetal (VII), das durch Hydrolyse nach oben beschriebenen Verfahren in IX überführt werden kann.

Beispiel 6 beschreibt den Einfluss von 2 Gew-% TMOF im Elektrolyten. Vollumsatz von III wird nach 333 Ah erreicht und liefert 2,4 g 4-tert-Butylbenzaldehyd (IX) sowie 320,8 g 4-tert-Butylbenzaldehyddimethylacetal (VII), das durch Hydrolyse nach oben beschriebenen Verfahren in IX überführt werden kann.

Beispiel 7 beschreibt den Einfluss von 3 Gew-% TMOF im Elektrolyten. Vollumsatz von III wird nach 306 Ah erreicht und liefert 0,8 g 4-tert-Butylbenzaldehyd (IX) sowie 334,5 g 4-tert-Butylbenzaldehyddimethylacetal (VII), das durch Hydrolyse nach oben beschriebenen Verfahren in IX überführt werden kann.

Beispiel 8 beschreibt den Einfluss von 3,5 Gew-% TMOF im Elektrolyten. Dies entspricht in etwa einem Moläquivalent bezogen auf die Menge des in der Elektrolyse eingesetzten 3-tert-Butylbenzaldehyds. Vollumsatz von III wird nach 293 Ah erreicht und liefert 0,6 g 4-tert-Butylbenzaldehyd (IX) sowie 338,0 g 4-tert-Butylbenzaldehyddimethylacetal (VII), das durch Hydrolyse nach oben beschriebenen Verfahren in IX überführt werden kann.

Beispiel 9 beschreibt den Einfluss von 4 Gew-% TMOF im Elektrolyten. Vollumsatz von III wird nach 288 Ah erreicht und liefert 0,7 g 4-tert-Butylbenzaldehyd (IX) sowie 337,5 g 4-tert-Butylbenzaldehyddimethylacetal (VII), das durch Hydrolyse nach oben beschriebenen Verfahren in IX überführt werden kann.

Beispiel 10 beschreibt den Einfluss von 4,5 Gew-% TMOF im Elektrolyten. Vollumsatz von III wird nach 279 Ah erreicht und liefert 0,6 g 4-tert-Butylbenzaldehyd (IX) sowie 344,1 g 4-tert-Butylbenzaldehyddimethylacetal (VII), das durch Hydrolyse nach oben beschriebenen Verfahren in IX überführt werden kann.

Beispiel 11 beschreibt den Einfluss von 5,5 Gew-% TMOF im Elektrolyten. Vollumsatz von III wird nach 279 Ah erreicht und liefert 0,6 g 4-tert-Butylbenzaldehyd (IX) sowie 339,2 g 4-tert-Butylbenzaldehyddimethylacetal (VII), das durch Hydrolyse nach oben beschriebenen Verfahren in IX überführt werden kann.

Beispiel 12 beschreibt die Verwendung eines zuvor über Molsieb getrockneten Elektrolyten in der elektrochemischen Methoxylierung. Der Elektrolyt wurde vor Beginn der Elektrolyse über Molsieb (30 g Molsieb pro 100 g Elektrolyt) der Firma Grace (Sylobead 564 C, 3 Ä, Aktivierung des Molsiebs bei 180°C/3mbar) getrocknet, bis ein Wassergehalt <0,05 Gew-% erreicht wurde. Das Molsieb wurde durch Filtration in einer Schutzgasatmosphäre aus dem Elektrolyten entfernt und dieser nach Zugabe von weiteren 9 g konzentrierter Schwefelsäure unter oben genannten Versuchsbedingungen umgesetzt. Vollumsatz von III wird nach 293 Ah erreicht und liefert 0,7 g 4-tert-Butylbenzaldehyd (IX) sowie 355,6 g 4-tert-Butylbenzaldehyddimethylacetal (VII), das durch Hydrolyse nach oben beschriebenen Verfahren in IX überführt werden kann.

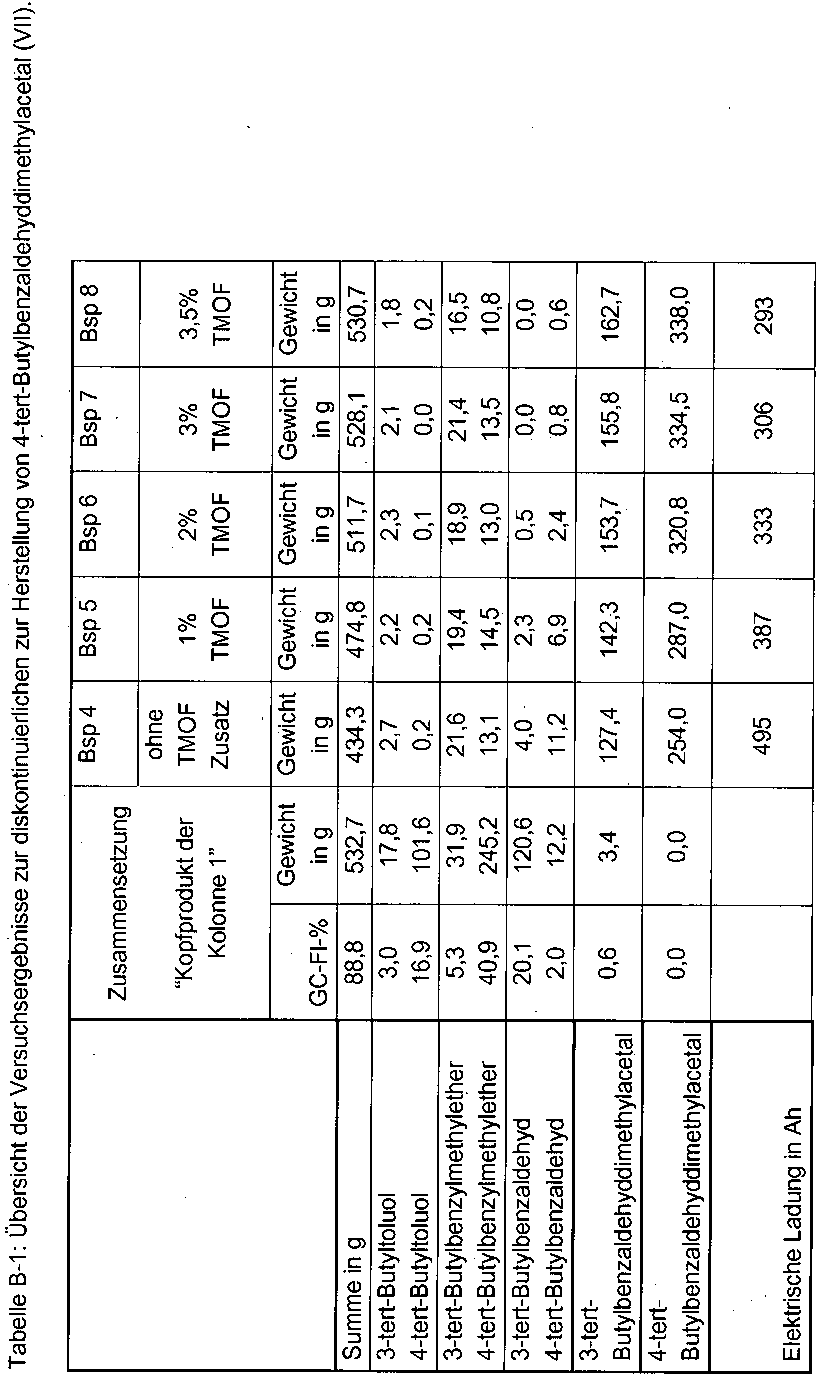

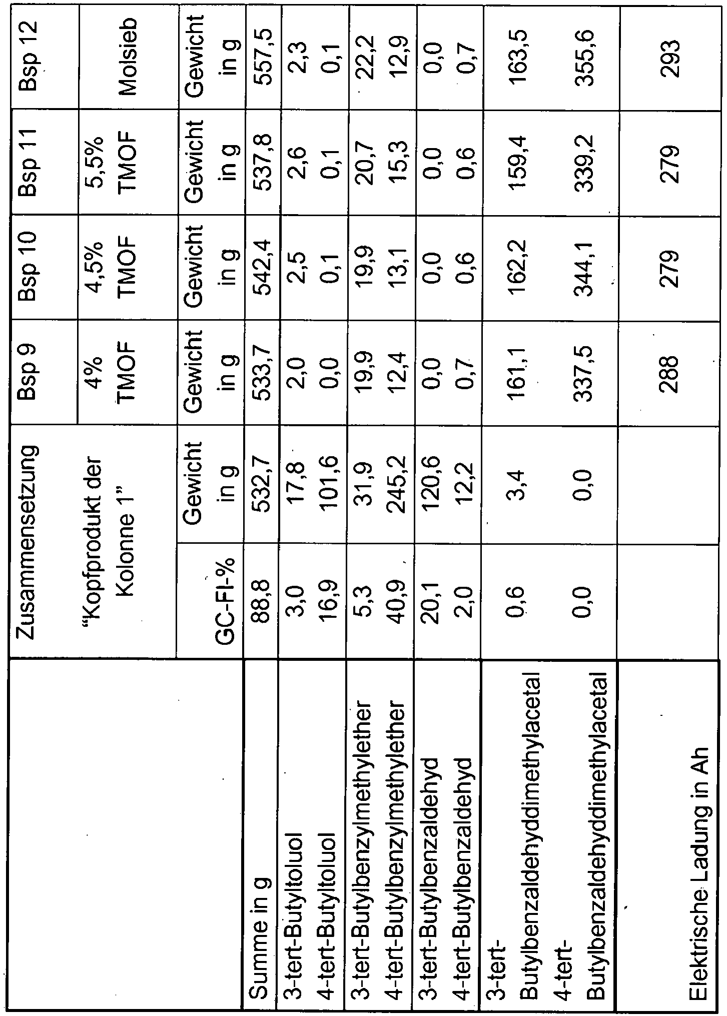

## Patentansprüche

1. Verfahren zur Herstellung von 2-Methyl-3-(4-tert-butylphenyl)-propanal der Formel I 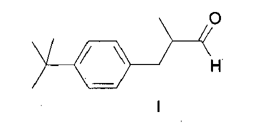 mit einem Gehalt von weniger als 0,3 Gew.-% 2-Methyl-3-(3-tert-butylphenyl)-propanal der Formel II 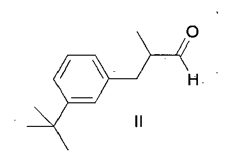 bezogen auf die Gesamtmasse der Verbindungen der Formeln I und II
ausgehend von 4-tert-Butyltoluol der Formel III 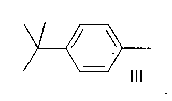 mit einen Gehalt von 0,5 bis 5 Gew.-% 3-tert-Butyltoluol der Formel IV 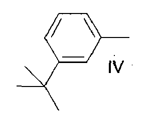 bezogen auf die Gesamtmasse der Verbindungen der Formeln III und IV als Ausgangsstoff
umfassend die Verfahrensschritte:
a) elektrochemische anodische Methoxylierung eines Gemisches enthaltend Verbindungen der Formeln III und IV sowie die als Zwischenprodukte anfallenden Benzylether der Formeln V und VI 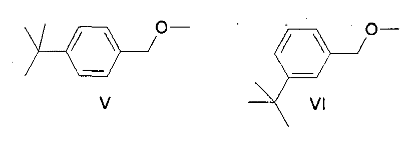 zu den Dimethylacetalen der Formeln VII und VIII 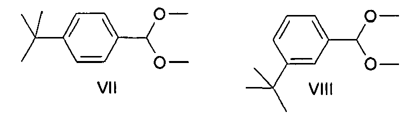 in einer Elektrolyselösung umfassend Methanol, mindestens ein Leitsalz sowie gegebenenfalls ein Cosolvens oder mehrere verschiedene Cosolventien;
b) Hydrolyse des in Verfahrensschritt a) gebildeten Gemisches der Dimethylacetale der Formeln VII und VIII zu den entsprechenden Benzaldehyden der Formeln IX und X 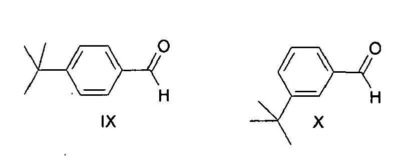 gefolgt von einer Abreicherung des Benzaldehyds der Formel X mittels Destillation;
wobei die Destillation in Verfahrensschritt b) zweistufig durchgeführt wird, indem in einer ersten Destillation mittels einer 1. Destillationskolonne ein Destillat am Kopf der 1. Destillationskolonne abdestilliert wird, welches zu mehr als 80 Gew.-% eine Mischung enthält, die aus nicht umgesetzten Ausgangsprodukten der Formeln III und IV, sowie den als Zwischenprodukten in Verfahrensschritt a) anfallenden Benzylethern der Formeln V und VI und dem Benzaldehyd der Formel X 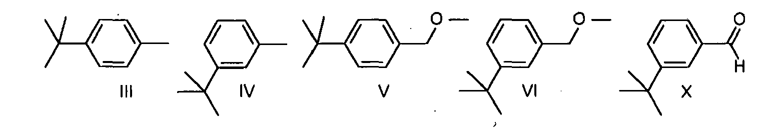 besteht,
und in einer zweiten Destillation mittels einer 2. Destillationskolonne der Benzaldehyd der Formel IX 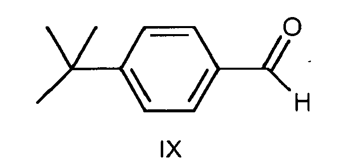 mit einem Gehalt von weniger als 0,5 Gew.-% des Benzaldehyds der Formel X bezogen auf die Gesamtmasse der Verbindungen der Formeln IX und X aus dem Sumpf der ersten Destillation am Kopf der 2.
Destillationskolonne abgetrennt wird, und wobei gegebenenfalls das am Kopf der 1. Destillationskolonne erhaltene Destillat, das die Verbindungen der Formeln III, IV, V, VI und X enthält, zusammen mit dem als Ausgangstoff eingesetzten 4-tert-Butyltoluol der Formel III wieder in Verfahrensschritt a) eingesetzt wird,
c) Umsetzung des in Verfahrensschritt b) erhaltenen Gemisches der Benzaldehyde der Formeln IX und X, wobei das Gemisch weniger als 0,3 Gew.-% des Benzaldehydes der Formel X enthält, mit Propanal unter basischen Bedingungen zu den Dehydrozimtaldehyden der Formeln XI und XII; 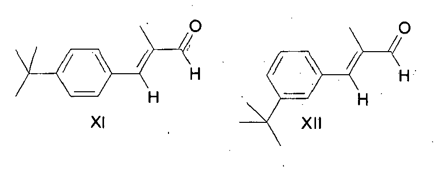
d) Katalytische Hydrierung der in Verfahrensschritt c) hergestellten Dehydrozimtaldehyde der Formeln XI und XII zu den Propanalen der Formeln I
und II, 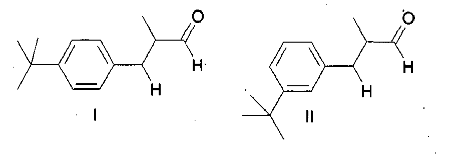 wobei der Gehalt des Propanals der Formel II weniger als 0,3 Gew.-% bezogen auf die Gesamtmasse der Verbindungen der Formeln I und II beträgt, gegebenenfalls gefolgt von einer Abtrennung von Lösungsmittelresten, Vorprodukten und Nebenprodukten mittels Destillation;
e) gegebenenfalls Reindestillation der in Verfahrensschritt d) gewonnenen Propanale der Formeln I und II zur Abreicherung des Propanals der Formel II.

2. Verfahren nach Anspruch 1, wobei das in Verfahrensschritt a) als Ausgangsstoff eingesetzte 4-tert-Butyltoluol der Formel III einen Gehalt von 2 bis 4 Gew.-% 3-tert-Butyltoluol der Formel IV bezogen auf die Gesamtmasse der Verbindungen der Formeln III und IV aufweist.

3. Verfahren nach Anspruch 1 oder 2, wobei das nach der Destillation in Verfahrensschritt b) erhaltene Gemisch der Benzaldehyde der Formeln IX und X einen Gehalt von weniger als 0,1 Gew.-% des Benzaldehydes der Formel X bezogen auf die Gesamtmasse der Verbindungen der Formeln IX und X aufweist.

4. Verfahren nach einem der Ansprüche 1 bis 3, wobei die Destillation in Verfahrensschritt b) zweistufig durchgeführt wird, indem in einer ersten Destillation mittels einer 1. Destillationskolonne ein Destillat am Kopf der 1. Destillationskolonne abdestilliert wird, welches zu mehr als 80 Gew.-% eine Mischung enthält, die aus nicht umgesetzten Ausgangsprodukten der Formeln III und IV, sowie den als Zwischenprodukten in Verfahrensschritt a) anfallenden Benzylethern der Formeln V und VI und dem Benzaldehyd der Formel X 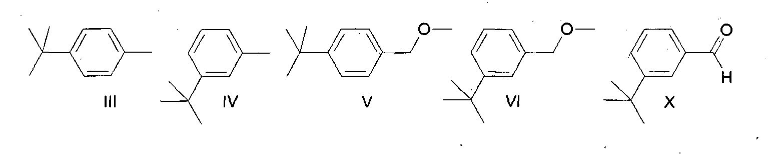 besteht,
und in einer zweiten Destillation mittels einer 2. Destillationskolonne der Benzaldehyd der Formel IX 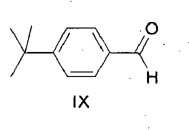 mit einem Gehalt von weniger als 0,1 Gew.-% des Benzaldehyds der Formel X bezogen auf die Gesamtmasse der Verbindungen der Formeln IX und X aus dem Sumpf der ersten Destillation am Kopf der 2. Destillationskolonne abgetrennt wird, und wobei gegebenenfalls das am Kopf der 1. Destillationskolonne erhaltene Destillat, das die Verbindungen der Formeln III, IV, V, VI und X enthält, zusammen mit dem als Ausgangstoff eingesetzten 4-tert-Butyltoluol der Formel III wieder in Verfahrensschritt a) eingesetzt wird.

5. Verfahren nach einem der Ansprüche 1 bis 4, wobei das nach der Destillation in Verfahrensschritt d) oder e) erhaltene Gemisch der Propanale der Formeln I und II einen Gehalt von weniger als 0,05 Gew.-% des Propanals der Formel II bezogen auf die Gesamtmasse der Verbindungen der Formeln I und II aufweist.

6. Verfahren zur Herstellung von 4-tert-Butylbenzaldehyd der Formel IX 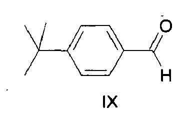 mit einen Gehalt von weniger als 0,1 Gew.-% 3-tert-Butylbenzaldehyd der Formel X 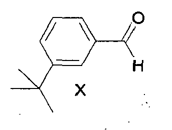 bezogen auf die Gesamtmasse der Verbindungen der Formeln IX und X, ausgehend von 4-tert-Butyltoluol der Formel III 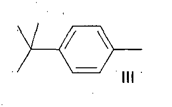 mit einem Gehalt von 0,5 bis 5 Gew.-% 3-tert-Butyltoluol der Formel IV 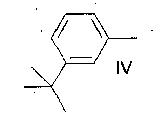 bezogen auf die Gesamtmasse der Verbindungen der Formeln III und IV als Ausgangsstoff
umfassend die Verfahrensschritte:
a) elektrochemische anodische Methoxylierung eines Gemisches enthaltend Verbindungen der Formeln III und IV sowie die als Zwischenprodukte anfallenden Benzylether der Formeln V und VI 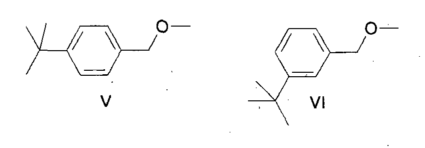 zu den Dimethylacetalen der Formeln VII und VIII 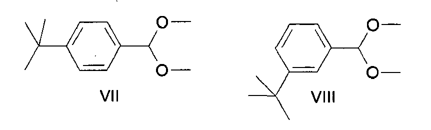 in einer Elektrolyselösung umfassend Methanol, mindestens ein Leitsalz sowie gegebenenfalls ein Cosolvens oder mehrere verschiedene Cosolventien;
b) Hydrolyse des in Verfahrensschritt a) gebildeten Gemisches der Dimethylacetale der Formeln VI und VIII zu den entsprechenden Benzaldehyden der Formeln IX und X 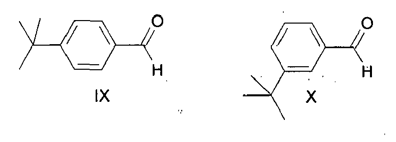 gefolgt von einer Abreicherung des Benzaldehyds der Formel X mittels Destillation,
wobei die Destillation in Verfahrensschritt b) zweistufig durchgeführt wird, indem in einer ersten Destillation mittels einer 1. Destillationskolonne ein Destillat am Kopf der 1. Destillationskolonne abdestilliert wird, welches zu mehr als 80 Gew.-% eine Mischung enthält, die aus nicht umgesetzten Ausgangsprodukten der Formeln III und IV, sowie den als Zwischenprodukten in Verfahrensschritt a) anfallenden Benzylethern der Formeln V und VI und dem Benzaldehyd der Formel X 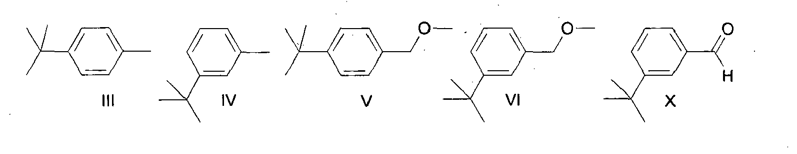 besteht,
und in einer zweiten Destillation mittels einer 2. Destillationskolonne der Benzaldehyd der Formel IX 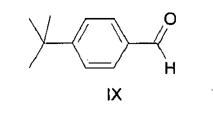 mit einem Gehalt von weniger als 0,5 Gew.-%, vorzugsweise weniger als 0,1 Gew.-% des Benzaldehyds der Formel X bezogen auf die Gesamtmasse der Verbindungen der Formeln IX und X aus dem Sumpf der ersten Destillation am Kopf der 2. Destillationskolonne abgetrennt wird, und wobei gegebenenfalls das am Kopf der 1..Destillationskolonne erhaltene Destillat, das die Verbindungen der Formeln III, IV, V, VI und X enthält, zusammen mit dem als Ausgangstoff eingesetzten 4-tert-Butyltoluol der Formel III wieder in Verfahrensschritt a) eingesetzt wird.

7. Verfahren nach Anspruch 6, wobei das am Kopf der 1. Destillationskolonne erhaltene Destillat zusammen mit dem als Ausgangstoff eingesetzten 4-tert-Butyltoluol der Formel III wieder in Verfahrensschritt a) eingesetzt wird.

8. Verfahren nach Anspruch 6 oder 7, wobei ein Anteil zwischen 2 und 7 Gew.-% des am Kopf der 1. Destillationskolonne erhaltenen Destillates verworfen wird, wobei der verworfenen Anteil des Destillates zu mehr als 20 Gew.-% aus dem Benzylether der Formel VI und dem Benzaldehyd der Formel X bezogen auf die Masse der Fraktion besteht.

9. Verfahren nach Anspruch 6, wobei der Benzaldehyd der Formel X aus dem Destillat, das am Kopf der 1. Destillationskolonne erhaltenen wird, nach chemischer Umwandlung in einen Feststoff von den weiteren flüssigen Bestandteilen des Destillates, insbesondere den Verbindungen der Formeln III, IV, V und VI, abgetrennt wird, und anschließend das weitgehend Aldehyd-freie aufgereinigte Destillat zusammen mit dem als Ausgangstoff eingesetzten 4-tert-Butyltoluol der Formel III wieder in Verfahrensschritt a) eingesetzt wird.

10. Verfahren nach Anspruch 9, wobei der Benzaldehyd der Formel X durch chemische Umwandlung zu einem bei Raumtemperatur festen Hydrazonderivat, Semicarbazonderivat, Oxim, Imin oder Bisulfit-Addukt umgesetzt wird.

11. Verfahren nach einem der Ansprüche 1 bis 5, wobei die Verfahrensschritte a) und b) nach einem der Ansprüche 6 bis 10 durchgeführt werden.

12. Verfahren zur Herstellung des Benzaldehyds der Formel X 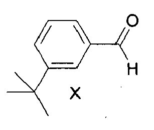 in einer Reinheit von mindestens 80 Gew.-% umfassend die Verfahrensschritte:
i) Chemische Umwandlung des Benzaldehyds der Formel X, der in dem Destillat enthalten ist, das am Kopf der 1. Destillationskolonne gemäß Anspruch 6 erhalten wird, in einen Feststoff;
ii) Abtrennung des in Verfahrensschritt i) gebildeten Feststoffes von den übrigen flüssigen Bestandteilen des Destillates, insbesondere den Verbindungen der Formeln III, ,IV, V und VI;
iii) Freisetzung des Benzaldehyds der Formel X aus dem in Verfahrensschritt ii) abgetrennten Feststoff.

13. Verfahren nach Anspruch 12, wobei in Verfahrensschritt i) der Benzaldehyd der Formel X zu einem bei Raumtemperatur festen Hydrazonderivat, Semicarbazonderivat, Oxim, Imin oder Bisulfit-Addukt umgesetzt wird, in Verfahrensschritt ii) das in Verfahrensschritt i) gebildete Hydrazonderivat, Semicarbazonderivat, Oxim, Imin oder Bisulfit-Addukt von den übrigen flüssigen Bestandteilen des Destillates, insbesondere den Verbindungen der Formeln III, IV, V und VI abgetrennt wird, und in Verfahrensschritt iii) aus dem in Verfahrensschritt ii) abgetrennten Hydrazonderivat, Semicarbazonderivat, Oxim, Imin oder Bisulfit-Addukt der Benzaldehyd der Formel X freigesetzt wird.

14. Verfahren nach einem der Ansprüche 1 bis 11, wobei der Wassergehalt der Elektrolyselösung in Verfahrensschritt a) kleiner gleich 20 Gew.-%, insbesondere kleiner gleich 10 Gew.-%, vorzugsweise kleiner gleich 5 Gew.-%, insbesondere kleiner gleich 0,05 Gew.-% beträgt.

15. Verfahren nach einem der Ansprüche 1 bis 11, wobei die Elektrolyselösung in Verfahrensschritt a) 0,1 bis 10 Gew.-% eines Orthoesters oder Mischungen veschiedener Orthoester enthält, wobei vorzugsweise als Orthoester Orthöameisensäureester und/oder Orthoessigsäureester eingesetzt werden.

## Claims

1. A process for preparing 2-methyl-3-(4-tert-butylphenyl)propanal of the formula I 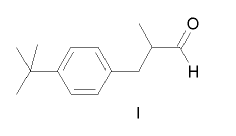 with less than 0.3% by weight content of 2-methyl-3-(3-tert-butylphenyl) propanal of the formula II 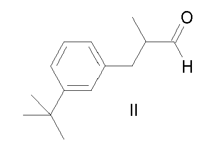 based on the total mass of the compounds of the formulae I and II
starting from 4-tert-butyltoluene of the formula III 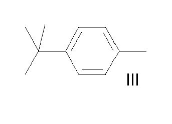 with from 0.5 to 5% by weight content of 3-tert-butyltoluene of the formula IV 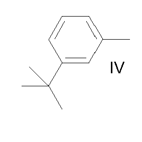 based on the total mass of the compounds of the formulae III and IV, as starting material,
comprising the steps of:
a) electrochemical anodic methoxylation of a mixture comprising compounds of the formulae III and IV, and also comprising the benzyl ethers of the formulae V and VI which are produced as intermediates 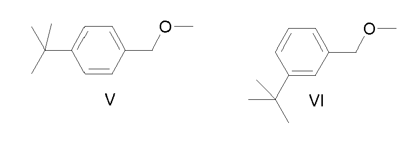 to give the dimethyl acetals of the formulae VII and VIII 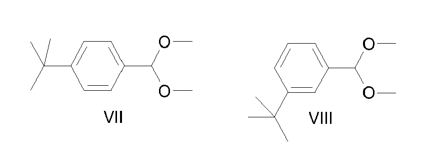 in an electrolysis solution comprising methanol, at least one conducting salt, and also optionally one cosolvent or a plurality of various cosolvents;
b) hydrolysis of the mixture of the dimethyl acetals of the formulae VII and VIII formed in step a) to give the corresponding benzaldehydes of the formulae IX and X 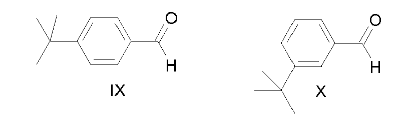 and then use of distillation to reduce the concentration of the benzaldehyde of the formula X;
where the distillation in step b) is carried out in two stages, by using a 1st distillation column in a first distillation to remove, at the top of the 1st distillation column, a distillate which comprises more than 80% by weight of a mixture composed of unreacted starting materials of the formulae III and IV, and also of the benzyl ethers of the formulae V and VI produced as intermediates in step a), and of the benzaldehyde of the formula X, 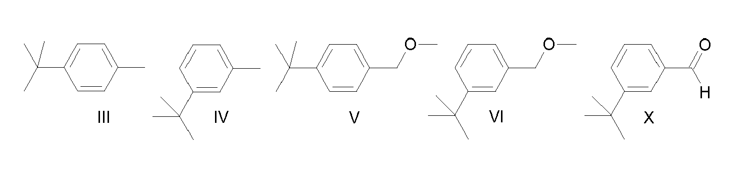 and using a 2nd distillation column in a second distillation to remove, at the top of the 2nd distillation column, the benzaldehyde of the formula IX 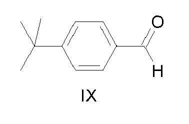 with less than 0.5% by weight content of the benzaldehyde of the formula X, based on the total mass of the compounds of the formulae IX and X, from the bottom product of the first distillation, and where optionally the distillate which is obtained at the top of the 1st distillation column and which comprises the compounds of the formulae III, IV, V, VI, and X is reused in step a) together with the 4-tert-butyltoluene of the formula III that is used as starting material,
c) reaction of the mixture of the benzaldehydes of the formulae IX and X obtained in step b), where the mixture comprises less than 0.3% by weight of the benzaldehyde of the formula X, with propanal under basic conditions to give the dehydrocinnamaldehydes of the formulae XI and XII; 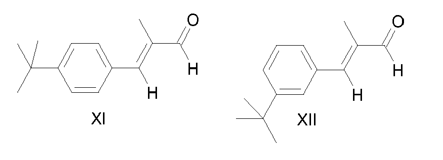
d) catalytic hydrogenation of the dehydrocinnamaldehydes of the formulae XI and XII prepared in step c) to give the propanals of the formulae I and II, 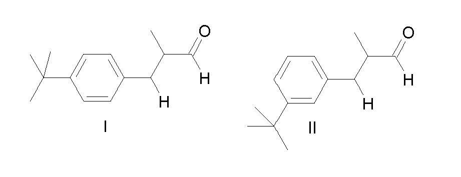 where the content of the propanal of the formula II is less than 0.3% by weight, based on the total mass of the compounds of the formulae I and II, optionally with subsequent use of distillation to remove solvent residues, precursors, and byproducts;
e) optionally final distillation of the propanals of the formulae I and II obtained in step d), in order to reduce the concentration of the propanal of the formula II.

2. The process according to claim 1, where the 4-tert-butyltoluene of the formula III used as starting material in step a) has from 2 to 4% by weight content of 3-tert-butyltoluene of the formula IV, based on the total mass of the compounds of the formulae III and IV.

3. The process according to claim 1 or 2, where the mixture of the benzaldehydes of the formulae IX and X obtained after the distillation in step b) has less than 0.1% by weight content of the benzaldehyde of the formula X, based on the total mass of the compounds of the formulae IX and X.

4. The process according to any of claims 1 to 3, where the distillation in step b) is carried out in two stages, by using a 1st distillation column in a first distillation to remove, at the top of the 1st distillation column, a distillate which comprises more than 80% by weight of a mixture composed of unreacted starting materials of the formulae III and IV, and also of the benzyl ethers of the formulae V and VI produced as intermediates in step a), and of the benzaldehyde of the formula X, 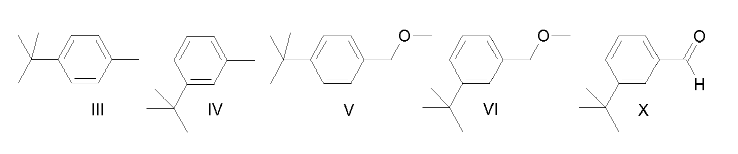 and using a 2nd distillation column in a second distillation to remove, at the top of the 2nd distillation column, the benzaldehyde of the formula IX 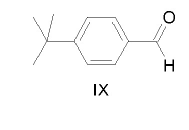 with less than 0.1% by weight content of the benzaldehyde of the formula X, based on the total mass of the compounds of the formulae IX and X, from the bottom product of the first distillation, and where optionally the distillate which is obtained at the top of the 1st distillation column and which comprises the compounds of the formulae III, IV, V, VI, and X is reused in step a) together with the 4-tert-butyltoluene of the formula III that is used as starting material.

5. The process according to any of claims 1 to 4, where the mixture of the propanals of the formulae I and II obtained after the distillation in step d) or e) has less than 0.05% by weight content of the propanal of the formula II, based on the total mass of the compounds of the formulae I and II.

6. A process for preparing 4-tert-butylbenzaldehyde of the formula IX 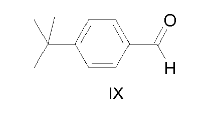 with less than 0.1% by weight content of 3-tert-butylbenzaldehyde of the formula X 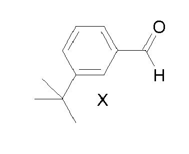 based on the total mass of the compounds of the formulae IX and X,
starting from 4-tert-butyltoluene of the formula III 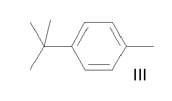 with from 0.5 to 5% by weight content of 3-tert-butyltoluene of the formula IV 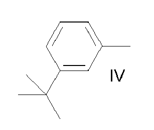 based on the total mass of the compounds of the formulae III and IV, as starting material,
comprising the steps of:
a) electrochemical anodic methoxylation of a mixture comprising compounds of the formulae III and IV, and also comprising the benzyl ethers of the formulae V and VI which are produced as intermediates 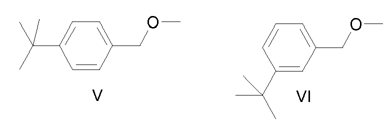 to give the dimethyl acetals of the formulae VII and VIII 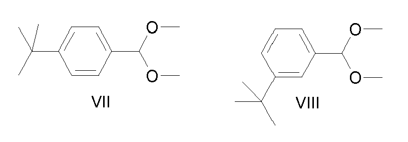 in an electrolysis solution comprising methanol, at least one conducting salt, and also optionally one cosolvent or a plurality of various cosolvents;
b) hydrolysis of the mixture of the dimethyl acetals of the formulae VII and VIII formed in step a) to give the corresponding benzaldehydes of the formulae IX and X 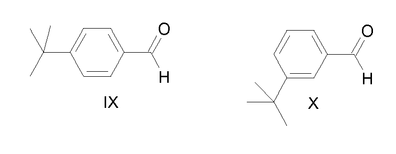 and then use of distillation to reduce the concentration of the benzaldehyde of the formula X;
where the distillation in step b) is carried out in two stages,
by using a 1st distillation column in a first distillation to remove, at the top of the 1st distillation column, a distillate which comprises more than 80% by weight of a mixture composed of unreacted starting materials of the formulae III and IV, and also of the benzyl ethers of the formulae V and VI produced as intermediates in step a), and of the benzaldehyde of the formula X, 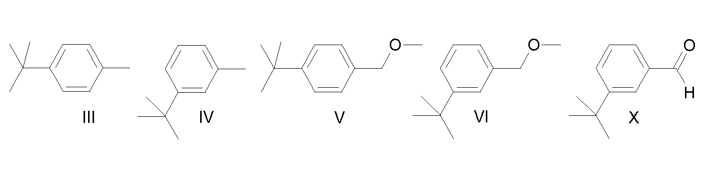 and using a 2nd distillation column in a second distillation to remove, at the top of the 2nd distillation column, the benzaldehyde of the formula IX 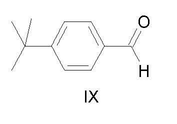 with less than 0.5% by weight, preferably less than 0.1% by weight, content of the benzaldehyde of the formula X, based on the total mass of the compounds of the formulae IX and X, from the bottom product of the first distillation, and where optionally, the distillate which is obtained at the top of the 1st distillation column and which comprises the compounds of the formulae III, IV, V, VI, and X, is reused in step a) together with the 4-tert-butyltoluene of the formula III that is used as starting material.

7. The process according to claim 6, where the distillate obtained at the top of the 1st distillation column is reused in step a) together with the 4-tert-butyltoluene of the formula III that is used as starting material.

8. The process according to claim 6 or 7, where a proportion of from 2 to 7% by weight of the distillate obtained at the top of the 1st distillation column is discarded, where the discarded proportion of the distillate is composed of more than 20% by weight of the benzyl ether of the formula VI and of the benzaldehyde of the formula X, based on the mass of the fraction.

9. The process according to claim 6, where, after chemical conversion to a solid, the benzaldehyde of the formula X from the distillate obtained at the top of the 1st distillation column is removed from the other liquid constituents of the distillate, in particular from the compounds of the formulae III, IV, V, and VI, and then the substantially aldehyde-free purified distillate is reused in step a) together with the 4-tert-butyltoluene of the formula III that is used as starting material.

10. The process according to claim 9, where the benzaldehyde of the formula X is converted by chemical conversion to a bisulfite adduct, imine, oxime, semicarbazone derivative, or hydrazone derivative solid at room temperature.

11. The process according to any of claims 1 to 5, where steps a) and b) are carried out according to any of claims 6 to 10.

12. A process for preparing the benzaldehyde of the formula X 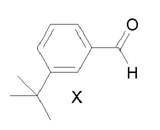 in at least 80% by weight purity, comprising the steps of:
i) chemical conversion of the benzaldehyde of the formula X which is comprised within the distillate obtained at the top of the 1st distillation column according to claim 6, into a solid;
ii) removal of the solid formed in step i) from the other liquid constituents of the distillate, in particular the compounds of the formulae III, IV, V, and VI;
iii) liberation of the benzaldehyde of the formula X from the solid removed in step ii).

13. The process according to claim 12, where, in step i), the benzaldehyde of the formula X is converted to a bisulfite adduct, imine, oxime, semicarbazone derivative, or hydrazone derivative solid at room temperature, the hydrazone derivative, semicarbazone derivative, oxime, imine, or bisulfite adduct formed in step i) is removed, in step ii), from the other liquid constituents of the distillate, in particular from the compounds of the formulae III, IV, V, and VI, and, in step iii), the benzaldehyde of the formula X is liberated from the hydrazone derivative, semicarbazone derivative, oxime, imine, or bisulfite adduct removed in step ii) .

14. The process according to any of claims 1 to 11, where the water content of the electrolysis solution in step a) is at most 20% by weight, in particular at most 10% by weight, preferably at most 5% by weight, in particular at most 0.05% by weight.

15. The process according to any of claims 1 to 11, where the electrolysis solution in step a) comprises from 0.1 to 10% by weight of an orthoester or mixture of various orthoesters, where orthoesters used preferably comprise orthoformic esters and/or orthoacetic esters.

## Revendications

1. Procédé pour la préparation de 2-méthyl-3-(4-tert-butylphényl)-propanal de formule I 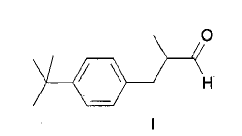 présentant une teneur inférieure à 0,3% en poids de 2-méthyl-3-(3-tert-butylphényl)-propanal de formule II 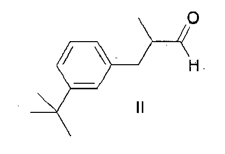 par rapport à la masse totale des composés de formule I et II, partant de 4-tert-butyltoluène de formule III 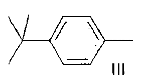 présentant une teneur de 0,5 à 5% en poids de 3-tert-butyltoluène de formule IV 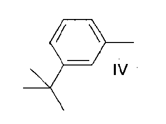 par rapport à la masse totale des composés de formule III et IV, comme substance de départ, comprenant les étapes de procédé de :
a) méthoxylation électrochimique anodique d'un mélange contenant des composés des formules III et IV ainsi que les benzyléthers des formules V et VI produits comme produits intermédiaires 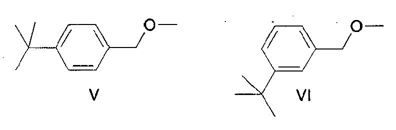 en diméthylacétals des formules VII et VIII 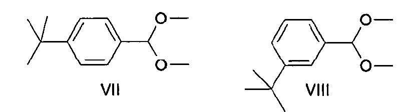 dans une solution d'électrolyse comprenant du méthanol, au moins un sel conducteur ainsi que le cas échéant un cosolvant ou plusieurs cosolvants différents ;
b) hydrolyse du mélange des diméthylacétals des formules VII et VIII, formé dans l'étape de procédé a), en benzaldéhydes correspondants des formules IX et X 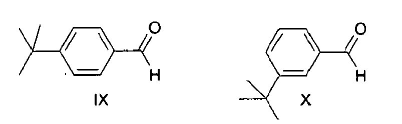 suivie d'un épuisement du benzaldéhyde de formule X par distillation ;
la distillation dans l'étape de procédé b) étant réalisée en deux étapes en ce que, dans une première distillation au moyen d'une première colonne de distillation, on élimine par distillation un distillat en tête de la première colonne de distillation qui contient, à raison de plus de 80% en poids, un mélange qui est constitué des produits de départ des formules III et IV non transformés ainsi que des benzyléthers des formules V et VI produits comme produits intermédiaires dans l'étape de procédé a) et du benzaldéhyde de formule X 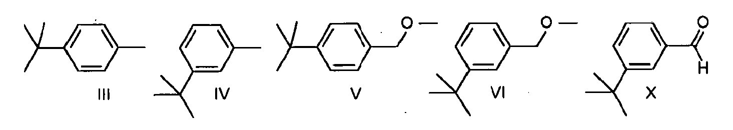 et, dans une deuxième distillation au moyen d'une deuxième colonne de distillation, on sépare le benzaldéhyde de formule IX 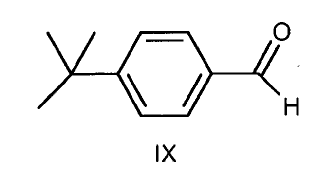 présentant une teneur inférieure à 0,5% en poids du benzaldéhyde de formule X, par rapport à la masse totale des composés des formules IX et X, du fond de la première distillation en tête de la deuxième colonne de distillation, et le distillat le cas échéant obtenu en tête de la première colonne de distillation, qui contient les composés des formules III, IV, V, VI et X, ensemble avec le 4-tert-butyltoluène de formule III utilisé comme substance de départ, étant à nouveau utilisé dans l'étape de procédé a),
c) transformation du mélange des benzaldéhydes des formules IX et X, obtenu dans l'étape de procédé b), le mélange contenant moins de 0,3% en poids du benzaldéhyde de formule X, avec du propanal dans des conditions basiques en aldéhydes déshydrocinnamiques des formules XI et XII ; 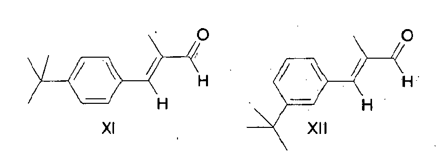
d) hydrogénation catalytique des aldéhydes déshydrocinnamiques des formules XI et XII préparés dans l'étape de procédé c) en propanals des formules I et II 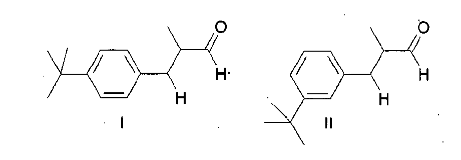 la teneur du propanal de formule II étant inférieure à 0,3% en poids par rapport à la masse totale des composés des formules I et II, le cas échéant suivie d'une séparation de restes de solvant, de précurseurs et de produits secondaires par distillation ;
e) le cas échéant distillation pure des propanals des formules I et II obtenus dans l'étape de procédé d) pour l'épuisement du propanal de formule II.

2. Procédé selon la revendication 1, le 4-tert-butyltoluène de formule III, utilisé comme substance de départ dans l'étape de procédé a), présentant une teneur de 2 à 4% en poids de 3-tert-butyltoluène de formule IV par rapport à la masse totale des composés des formules III et IV.

3. Procédé selon la revendication 1 ou 2, le mélange des benzaldéhydes des formules IX et X, obtenu après la distillation dans l'étape de procédé b), présentant une teneur inférieure à 0,1% en poids du benzaldéhyde de formule X, par rapport à la masse totale des composés des formules IX et X.

4. Procédé selon l'une quelconque des revendications 1 à 3, la distillation dans l'étape de procédé b) étant réalisée en deux étapes en ce que, dans une première distillation au moyen d'une première colonne de distillation, on élimine par distillation un distillat en tête de la première colonne de distillation qui contient, à raison de plus de 80% en poids, un mélange qui est constitué des produits de départ des formules III et IV non transformés ainsi que des benzyléthers des formules V et VI produits comme produits intermédiaires dans l'étape de procédé a) et du benzaldéhyde de formule X 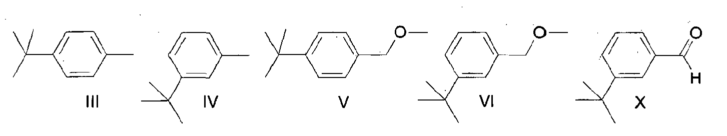 et, dans une deuxième distillation au moyen d'une deuxième colonne de distillation, on sépare le benzaldéhyde de formule IX 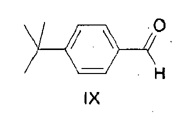 présentant une teneur inférieure à 0,1% en poids du benzaldéhyde de formule X, par rapport à la masse totale des composés des formules IX et X, du fond de la première colonne de distillation en tête de la deuxième colonne de distillation, et le distillat le cas échéant obtenu en tête de la première colonne de distillation, qui contient les composés des formules III, IV, V, VI et X, ensemble avec le 4-tert-butyltoluène de formule III utilisé comme substance de départ, étant à nouveau utilisé dans l'étape de procédé a).

5. Procédé selon l'une quelconque des revendications 1 à 4, le mélange des propanals des formules I et II, obtenu après la distillation dans l'étape de procédé d) ou e), présentant une teneur inférieure à 0,05% en poids du propanal de formule II par rapport à la masse totale des composés des formules I et II.

6. Procédé pour la préparation de 4-tert-butylbenzaldéhyde de formule IX 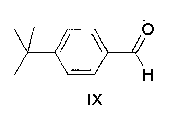 présentant une teneur inférieure à 0,1% en poids de 3-tert-butylbenzaldéhyde de formule X 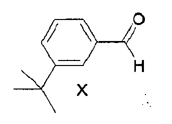 par rapport à la masse totale des composés des formules IX et X, partant de 4-tert-butyltoluène de formule III 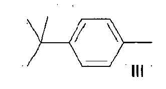 présentant une teneur de 0,5 à 5% en poids de 3-tert-butyltoluène de formule IV 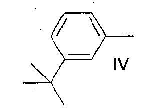 par rapport à la masse totale des composés des formules III et IV, comme substance de départ, comprenant les étapes de procédé de :
a) méthoxylation électrochimique anodique d'un mélange contenant des composés des formules III et IV ainsi que les benzyléthers des formules V et VI produits comme produits intermédiaires 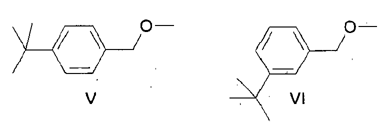 en diméthylacétals des formules VII et VIII 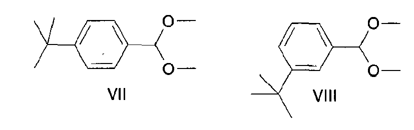 dans une solution d'électrolyse comprenant du méthanol, au moins un sel conducteur ainsi que le cas échéant un cosolvant ou plusieurs cosolvants différents ;
b) hydrolyse du mélange des diméthylacétals des formules VII et VIII, formé dans l'étape de procédé a), en benzaldéhydes correspondants des formules IX et X 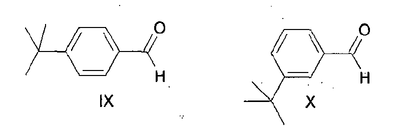 suivie d'un épuisement du benzaldéhyde de formule X par distillation ;
la distillation dans l'étape de procédé b) étant réalisée en deux étapes en ce que, dans une première distillation au moyen d'une première colonne de distillation, on élimine par distillation un distillat en tête de la première colonne de distillation qui contient, à raison de plus de 80% en poids, un mélange qui est constitué des produits de départ des formules III et IV non transformés ainsi que des benzyléthers des formules V et VI produits comme produits intermédiaires dans l'étape de procédé a) et du benzaldéhyde de formule X 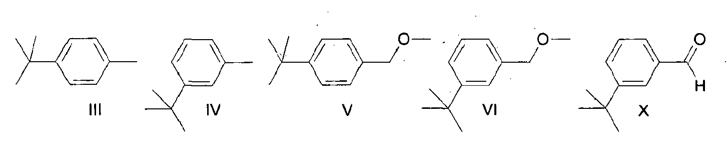 et, dans une deuxième distillation au moyen d'une deuxième colonne de distillation, on sépare le benzaldéhyde de formule IX 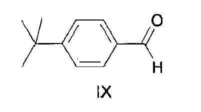 présentant une teneur inférieure à 0,5% en poids, de préférence inférieure à 0,1% en poids, du benzaldéhyde de formule X, par rapport à la masse totale des composés des formules IX et X, du fond de la première distillation en tête de la deuxième colonne de distillation, et le distillat le cas échéant obtenu en tête de la première colonne de distillation, qui contient les composés des formules III, IV, V, VI et X, ensemble avec le 4-tert-butyltoluène de formule III utilisé comme substance de départ, étant à nouveau utilisé dans l'étape de procédé a).

7. Procédé selon la revendication 6, le distillat obtenu en tête de la première colonne de distillation étant à nouveau utilisé ensemble avec le 4-tert-butyltoluène de formule III dans l'étape de procédé a).

8. Procédé selon la revendication 6 ou 7, une partie entre 2 et 7% en poids du distillat obtenu en tête de la première colonne de distillation étant rejetée, la partie rejetée du distillat étant constituée, à raison de plus de 20% en poids, du benzyléther de formule VI et du benzaldéhyde de formule X par rapport à la masse de la fraction.

9. Procédé selon la revendication 6, le benzaldéhyde de formule X étant séparé du distillat, qui est obtenu en tête de la première colonne de distillation, après transformation chimique en un solide, des autres constituants liquides du distillat, en particulier des composés des formules III, IV, V et VI, et le distillat purifié, dans une large mesure exempt d'aldéhyde, étant ensuite à nouveau utilisé, ensemble avec le 4-tert-butyltoluène de formule III utilisé comme substance de départ, dans l'étape de procédé a).

10. Procédé selon la revendication 9, le benzaldéhyde de formule X étant transformé par transformation chimique en un dérivé d'hydrazone, un dérivé de semi-carbazone, une oxime, une imine ou un produit d'addition de bisulfite, solide à température ambiante.

11. Procédé selon l'une quelconque des revendications 1 à 5, les étapes de procédé a) et b) étant réalisées selon l'une quelconque des revendications 6 à 10.

12. Procédé pour la préparation du benzaldéhyde de formule X 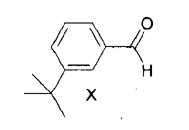 en une pureté d'au moins 80% en poids, comprenant les étapes de procédé de :
i) transformation chimique du benzaldéhyde de formule X, qui est contenu dans le distillat obtenu en tête de la première colonne de distillation selon la revendication 6, en un solide ;
ii) séparation du solide formé dans l'étape de procédé i) des autres constituants liquides du distillat, en particulier des composés des formules III, IV, V et VI ;
iii) libération du benzaldéhyde de formule X du solide séparé dans l'étape de procédé ii).

13. Procédé selon la revendication 12, le benzaldéhyde de formule X étant transformé, dans l'étape de procédé i), en un dérivé d'hydrazone, un dérivé de semi-carbazone, une oxime, une imine ou un produit d'addition de bisulfite, solide à température ambiante ; dans l'étape de procédé ii), le dérivé d'hydrazone, le dérivé de semi-carbazone, l'oxime, l'imine ou le produit d'addition de bisulfite formé dans l'étape de procédé i) étant séparé des autres constituants liquides du distillat, en particulier des composés des formules III, IV, V et VI ; et, dans l'étape iii), le benzaldéhyde de formule X étant libéré du dérivé d'hydrazone, du dérivé de semi-carbazone, de l'oxime, de l'imine ou du produit d'addition de bisulfite séparé dans l'étape de procédé ii).

14. Procédé selon l'une quelconque des revendications 1 à 11, la teneur en eau de la solution d'électrolyse dans l'étape de procédé a) étant inférieure à 20% en poids, en particulier inférieure à 10% en poids, de préférence inférieure à 5% en poids, en particulier inférieure à 0,05% en poids.

15. Procédé selon l'une quelconque des revendications 1 à 11, la solution d'électrolyse dans l'étape de procédé a) contenant 0,1 à 10% en poids d'un orthoester ou de mélanges de différents orthoesters, des esters de l'acide orthoformique et/ou des esters de l'acide orthoacétique étant de préférence utilisés comme orthoester.
